# EUROPEAN PATENT SPECIFICATION

(11) **EP 3 867 635 B1**
(45) Date of publication and mention of the grant of the patent: **18.02.2026**
(21) Application number: 19873333.9
(22) Date of filing: 17.10.2019
(51) Int. Cl.: G01N 29/00, A61B 5/157, B01L 3/00, G01N 1/40, G01N 1/28, A61B 5/15

(54) **EXTERNAL SONICATION SYSTEM**
EXTERNES BESCHALLUNGSSYSTEM
SYSTÈME DE SONICATION EXTERNE

(30) Priority: 17.10.2018 US 201862747045 P
(43) Date of publication of application: 25.08.2021
(73) Proprietor: Hemex Health, Inc., Portland, OR 97239 (US)
(72) Inventor: GALEN, Peter, Portland, OR 97239 (US); BERNSTEIN, Oren, Portland, OR 97239 (US); BLEDSOE, James Daren, Portland, OR 97239 (US); GRUPP, Daniel E., Portland, OR 97239 (US); HOYT, Joshua King, Portland, OR 97239 (US); SAYLER, David John, Portland, OR 97239 (US); THORNE, James, Portland, OR 97239 (US); WIKANDER, Jered, Portland, OR 97239 (US); WITTE, Tyler, Portland, OR 97239 (US)
(74) Representative: Tombling, Adrian George
(86) International application number: PCT/US2019/056832
(87) International publication number: WO 2020/081876

(56) References cited:
- EP-A1- 1 146 347
- EP-B1- 1 879 688
- WO-A1-2005/111614
- WO-A1-2005/111614
- GB-A- 2 403 729
- US-A- 5 468 616
- US-A- 5 853 994
- US-A1- 2002 009 015
- US-A1- 2003 234 642
- US-A1- 2007 055 161
- US-A1- 2013 289 371
- US-A1- 2013 289 371
- US-A1- 2014 001 058
- US-A1- 2014 001 058
- US-A1- 2016 030 007
- US-A1- 2016 216 284
- US-A1- 2016 216 284
- US-A1- 2018 064 384
- US-A1- 2018 224 472
- US-B2- 6 984 989

## Description

### CROSS-REFERENCE TO RELATED APPLICATIONS

This application claims the benefit of pending U.S. Provisional Patent Application Serial No. 62/747,045, filed October 17, 2018.

### BACKGROUND

Patient diagnostic services save lives, reduce the time to treatment for the patient and provide valuable insight for targeted treatment. In many developed countries, modem medical facilities can provide patients with the most advanced diagnostic services allowing patients to be efficiently and effectively treated. In less developed countries or regions, high quality medical facilities and diagnostic services can be lacking, often due to economic and infrastructure considerations. In many less developed countries, the economy cannot afford the latest in medical technology and infrastructure, such as a robust power grid or highly trained clinicians, required to support the high demands of modem medical technology. Sadly, a large portion of the world's population resides in underserved or underdeveloped areas where the lack of efficient and effective diagnostic services critically impacts the population morbidity, mortality and overall health. This lack of medical care can lead or contribute to knock-on effects, such as low economic and educational development.

Often, many less developed countries and areas also lack sufficient trained users that are typically required to perform the necessary diagnostic services. This can lead to inconclusive or erroneous results from diagnostic services or to significant delays in diagnosis as the diagnostic services are required to be performed in another location that has the requisite infrastructure and/or knowledge to perform the diagnostic service. For patients, this can mean further delays in treatment, which can decrease their chances of survival, increase the spread of the disease, and/or lead to increased debilitation caused by the disease or condition.

Where large laboratories may be prohibitively expensive and difficult to staff, point-of-care diagnostic devices may provide an effective solution. Such a solution could provide timely, accurate, and cost-effective health care.

One of the treatable common ailments effecting less developed countries are hemoglobin disorders, such as sickle cell disease (SCD), thalassemia and other hemoglobinopathies. These are genetic disorders that are believed to have evolved in response to malaria. With population migration, these conditions have spread to the global population and affect the livelihood and health of a large number of people. With early detection or diagnosis, these conditions can be treated and managed before they have significant adverse impact on the stricken individual. As with malaria, these disorders affect the populations of less developed countries and areas, which have limited to no access to the diagnostic services to rapidly, effectively and efficiently diagnose the conditions.

A further challenge is unreliable power sources in less developed areas. Thus it would be desirable to have diagnostic solutions that are of low enough power consumption to be able to run on batteries.

Yet a further challenge is a point-of-care device is subject to varying conditions of the environment, the patient sample, and disposable elements of the device itself. This may yield wide variation in test results. Thus it is desirable to have a diagnostic device that is either insensitive to such variations or is self-calibrating.

Yet a further challenge is current sonication implementations to disrupt components of the sample. Current methods include direct sonication (i.e., a sonication probe is immersed into and in contact with the sample) and wet sonication (i.e., sonication bath where the sample is placed in a container and then partially immersed in an ultrasonic bath). Each implementation has its own drawbacks-some of which include sample size, lack of energy focus, potential contamination issues, excessive heat, and the like. The volume or size of the sample directly impacts the amount of ultrasonic energy required for total disruption. The fluid viscosity also impacts energy transfer-more viscous samples require more ultrasonic energy which can result in heat generation. Excessive heat generated can damage or destroy the sample being tested.

Yet a further challenge is that some current detection systems do not account for samples having target materials which do not respond to an applied magnetic field, whether due to size, shape, too little magnetic response (i.e., material does not have enough magnetic components to respond to the applied magnetic field), no magnetic response (i.e., material has no magnetic components), or combinations thereof.

What is needed is a system or method for lysing or disrupting the sample in an effective manner, a system for processing a sample to collect as much diagnostically-relevant information as possible, and a system or device that is resistant to environmental condition. External sonication is effective in maximizing sample content disruption while optimizing energy transfer into the sample, minimizing heat generation in the sample, minimizing heat and energy dissipation external not into the sample. Additionally, target materials that are unresponsive to an applied magnetic field in their normal state can be processed so that the materials respond to the applied magnetic field, thereby obtaining diagnostically-relevant information which may have been missed or not collected.

What is further needed is a flexible seal, such as for a reader, can provide resistance to environmental conditions and external contaimination within detection system.

US 2018/224472 A1 discloses an external sonication system for disrupting a sample or component.

### BRIEF DESCRIPTION OF THE DRAWINGS

FIG. 1A illustrates an example diagnostics system.
FIG. 1B illustrates an example diagnostics system.
FIGS. 2A-2B illustrate example cartridges.
FIGS. 3A-3B illustrate an example magneto-optical detection (MOD) system.
FIG. 4 is an example analysis method using the example MOD system of FIGS. 3A-3B.
FIG. 5 is a block diagram of an example diagnostics system.
FIG. 6 is a block diagram of another example cartridge.
FIG. 7 illustrates an example patient sample analysis process.
FIGS. 8A-8D illustrate another example cartridge.
FIGS. 8E-8L illustrate an example cuvette of the example cartridge.
FIG. 9 illustrates an example reader network.
FIGS. 10A-10B illustrate a sample pre-sonication and post-sonication.
FIG. 11 is a block diagram of an example external sonicator system.
FIGS. 12A-12D illustrate example probe heads.
FIG. 13 is a block diagram of another example external sonicator system.
FIG. 14 is a block of an example driver.
FIGS. 15A-15B illustrate out-of-phase and in-phase waveforms for a resonate point.
FIGS. 16A-16B illustrate sonication waveforms.
FIG. 17 illustrates an example kit for non-magnetic material processing.
FIG. 18 illustrates an example stamper.

### DETAILED DESCRIPTION

Various example point-of-care, in vitro diagnostic devices and methods for detecting and helping to diagnose blood disorders, and specifically inherited blood disorders, infections and/or other conditions/diseases, are described herein. The disclosed diagnostic devices include a cartridge and a reader that interface to analyze a patient biologic sample, such as a blood sample, to provide a diagnosis, or data regarding one or more disorders, diseases, or conditions of the patient. The cartridge can contain or support the biologic sample for analysis by one or more diagnostic systems of the reader. Suitable devices, systems, and/or methods of point-of-care, in vitro diagnosis, detecting and helping to diagnose blood disorders, and specifically inherited blood disorders and/or other conditions/diseases may include those described in one or more of the following U.S. patents and published applications, 10,349,589; 10,375,909; 2018/0067101.

In the following description, the term "resonate point" is used to describe a point or location on or within a system or a point or location on or within a component of a system which produces resonance. The resonance can be a resonant frequency. The resonance can be electrical or mechanical.

In the following descriptions, the term "sample" is used to describe at least one material to undergo testing, processing, combinations thereof, or the like. The sample can be plant-based, organism-based, or animal-based. For example, the sample can be derived or obtained from a plant, algae, mineral, or the like. As another example, the sample, such as one derived or obtained from an organism, an animals, or a human, can a biological fluid, a biological semi-solid, a biological solid which can be liquefied in any appropriate manner, a suspension, a portion of the suspension, a component of the suspension, or the like. For the sake of convenience, the sample referenced is whole blood, though it should be understood that the method and system described and discussed herein is used with any appropriate sample, such as urine, blood, bone marrow, buffy coat, cystic fluid, ascites fluid, stool, semen, cerebrospinal fluid, nipple aspirate fluid, saliva, amniotic fluid, mucus membrane secretions, aqueous humor, vitreous humor, vomit, vaginal fluid, and any other physiological fluid or semi-solid. For example, the sample is a tissue sample or a material from adipose tissue, an adrenal gland, bone marrow, a breast, a caudate, a cerebellum, a cerebral cortex, a cervix, a uterus, a colon, an endometrium, an esophagus, a fallopian tube, a heart muscle, a hippocampus, a hypothalamus, a kidney, a liver, a lung, a lymph node, an ovary, a pancreas, a pituitary gland, a prostate, a salivary gland, a skeletal muscle, skin, a small intestine, a large intestine, a spleen, a stomach, a testicle, a thyroid gland, or a bladder.

In the following descriptions, the term "target material" is used to describe a material of interest within the sample. It should also be understood that the target material can be a fraction of a sample (e.g., plasma, buffy coat, or red blood cells) or a component of a sample (a cell, such as ova, fetal material (such as trophoblasts, nucleated red blood cells, fetal red blood cells, fetal white blood cells, fetal DNA, fetal RNA, or the like), a circulating tumor cell ("CTC"), a circulating endothelial cell, an immune cell (i.e. naive or memory B cells or naïve or memory T cells), a mesenchymal cell, a stem cell, a vesicle, such as an exosome, a liposome, a protein, a nucleic acid, a biological molecule, a naturally occurring or artificially prepared microscopic unit having an enclosed membrane, parasites (e.g. spirochetes; malaria-inducing agents, such as Plasmodium), microorganisms, viruses, or inflammatory cells.

In the following descriptions, the term "biomarker" is used to describe a substance that is present on or within the target material (i.e. intracellular or extracellular the target material; internalized, such as through phagocytosis, within the target material; or the like). Biomarkers include, but are not limited to, peptides, proteins, subunits, domains, motifs, epitopes, isoforms, DNA, RNA, crystals such as hemozoin, or the like. The biomarker may be a target molecule for drug delivery.

In the following descriptions, the term "affinity molecule" is used to describe any molecule (or group of molecules) that is capable of binding or interacting with a biomarker or directly to a portion of the target object such as to a virus, bacteria, infected cell, or the like. The interaction or binding can be covalent or non-covalent. The affinity molecule includes, but is not limited to, an antibody, a hapten, a protein, an aptamer, an oligonucleotide, a polynucleotide, or any appropriate molecule for interacting with or binding to the biomarker or directly to a portion of the target object such as to a virus, bacteria, infected cell, or the like.

In the following descriptions, the term "conjugate" is used to describe a first chemical, molecule, moiety, or the like bound to or interacted with a second chemical, molecule, moiety, or the like. The binding or interaction is direct or indirect. Direct binding or interaction includes covalent or non-covalent interactions between the biomarker and the detection moiety. Indirect binding or interaction includes the use of at least first and second complementary molecules which form binding pairs. The first and second complementary molecules are, in combination, binding pairs which binds or interacts in at least one of the following manners: hydrophobic interactions, ionic interactions, hydrogen bonding interactions, non-covalent interactions, covalent interactions, affinity interactions, or the like. The binding pairs include, but are not limited to, immune-type binding-pairs, such as, antigen-antibody, antigen-antibody fragment, hapten-anti-hapten, or primary antibody-secondary antibody; nonimmune-type binding-pairs, such as biotin-avidin, biotin-streptavidin, folic acid-folate binding protein, hormone-hormone receptor, lectin-specific carbohydrate, enzyme-enzyme, enzyme-substrate, enzyme-substrate analog, enzyme-pseudo-substrate (substrate analogs that cannot be catalyzed by the enzymatic activity), enzyme-cofactor, enzyme-modulator, enzyme-inhibitor, or vitamin B12-intrinsic factor. Other suitable examples of binding pairs include complementary nucleic acid fragments (including complementary nucleotides, oligonucleotides, or polynucleotides); Protein A-antibody; Protein G-antibody; nucleic acid-nucleic acid binding protein; polymeric linkers (e.g., polyethylene glycol); or polynucleotide-polynucleotide binding protein.

### Cartridge and MOD System

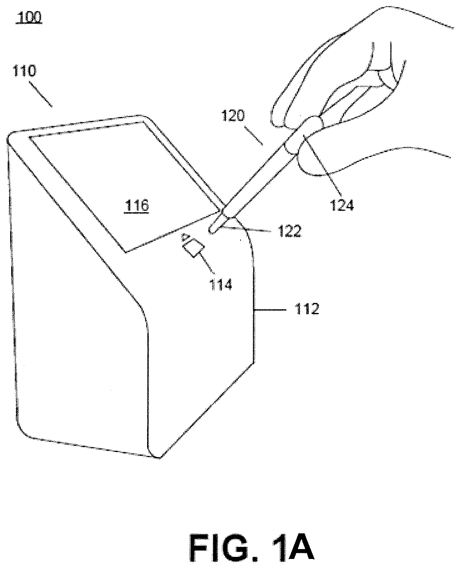

FIG. 1A illustrates an example reader 110 and cartridge 120 of a point-of-care blood diagnostic system 100. A point-of-care blood diagnostic system includes devices that can be physically located at the site at which patients are tested, though they can be used in a laboratory setting also, and sometimes treated to provide quick results and highly effective treatment. Point-of-care devices can provide information and help in diagnosing patient disorders and/or infections while the patient is present with potentially immediate referral and/or treatment. Unlike gold standard laboratory-based blood testing for disorders and/or infections, the disclosed point-of-care devices enable diagnosis close to the patient while maintaining high sensitivity and accuracy aiding efficient and effective early treatment of the disorder and/or infection.

The reader 110 includes a housing 112, a cartridge receptacle 114 and a display 116. The cartridge 120, which contains the patient sample and, optionally diluents, stains (e.g., fluorescent, chromogenic, or the like), reagents and/or materials, is inserted into the cartridge receptacle 114 of the reader 110 to transfer the patient sample, treated or untreated, into the reader 110 to perform a diagnostic test or analysis. The cartridge 120 more generally is placed in proximity to the reader in such a manner that the reader can interact with one or more elements of the cartridge to perform analysis of the patient sample. The cartridge 120 can include a pipette-like end 122 and a bulb 124 for siphoning a patient sample into the cartridge 120 in preparation for the diagnostic test. Alternatively, the cartridge 120 can include a capillary tube by which the patient sample can be obtained for analysis and/or testing. In a further embodiment, collection of the patient blood sample can be performed using blotting paper that is included in the cartridge 120 to collect a blood spot that can be analyzed by the point-of-care blood diagnostic system 100.

The housing 112 of the reader 110 can be constructed of materials such as plastic or metal and is preferably sealed with a smooth surface, which allows the reader 110 to be easily cleaned and/or disinfected and resist external water and or dust. Further, the housing 112 is sufficiently strong to allow the safe transport and use of the reader 110 without substantial damage to the reader 110 and the diagnostic systems within. Additionally, the housing 112 can have properties that shield or minimize the exposure of the interior of the reader 110 to temperature and/or humidity variations and/or light intrusion. The robustness of the reader 110 allows it to be used in a variety of locations and environments without adversely affecting the results of the diagnostic system.

The housing 112 of the reader 110 can also include vibration isolation to prevent vibration of the reader 110 during the measurement process to assist with preventing analysis error of the patient sample. Vibration isolation can include suspending and/or isolating the components and/or systems of the reader 110 within the housing 112 or containing the components and/or systems within an internal housing that is suspended and/or isolated from the external housing 112. Alternative vibration isolation can include anti-vibration feet or mounts on which the reader 110 can sit on a surface. Additional vibration isolation can include placing the reader 110 on a cushioned and/or anti-vibration mat to reduce or limit the vibration and/or disturbance of the reader 110 by its external environment.

The cartridge receptacle 114 can be conformably shaped to receive the cartridge 120. The cartridge 120 can be received partially or completely into the cartridge receptacle 114 or the reader 110. Alternatively, the cartridge 120 can be otherwise connected, such as by an external receptacle or conduit, to the reader 110 to transfer the patient sample, or portion thereof, into the reader 110. Such an external receptacle or conduit can be electrically coupled to the electronics housed in the reader 110 by a wireless or hard-wire connection of any suitable configuration.

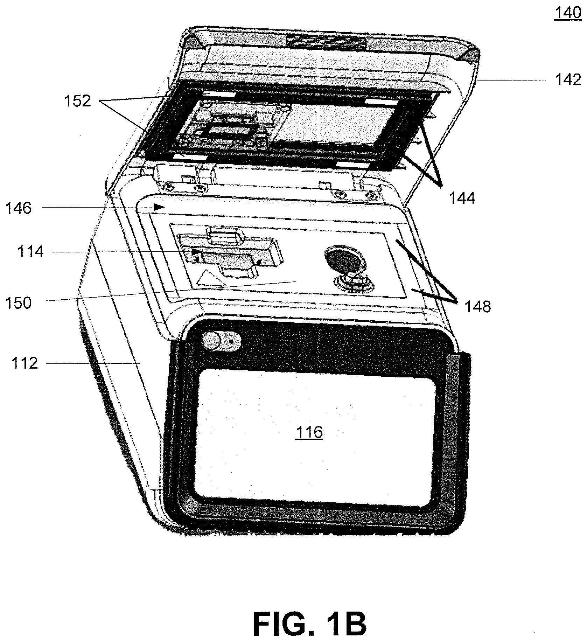

FIG. 1B illustrates an example reader 140. The reader 140 is similar to the reader 110, except the reader 140 includes a lid 142 hingedly connected to the housing 112, such that the lid 142 is connected to the housing 112 with a hinge. The hinge can be a butt hinge, a ball bearing hinge, a flush hinge, a hospital hinge, a strap hinge, a case hinge, an olive knuckle hinge, a pivot hinge, a heavy duty hinge, a double action spring, a butterfly hinge, a bi-fold hinge, a concealed hinge, a T-hinge, a barrel hinge, a continuous hinge, a flag hinge, a take apart hinge, or a knife hinge.

The reader 140 also includes a metal ring 148 and a flexible seal 144 including magnets 152, which secure the lid 142 to the housing 112 via the flexible seal 144 and the metal ring 148. The magnets 152 are located external to the flexible seal 144, such as on an outer surface of the flexible seal 144 proximal to the metal ring 148, or within the flexible seal 144, such as being housed within the flexible seal 144 in a location proximal to the metal ring 148. In one embodiment, the metal ring 148 is located on an upper surface 150 of the housing 112, and the flexible seal 144 is attached to a first surface of the lid 142. In another embodiment, the metal ring 148 is located on the first surface of the lid 142, and the flexible seal 144 is attached to the upper surface 150 of the housing. The flexible seal 144 can be fixedly, removably, or releasably attached to the lid 142 or the housing 112.

Though reader 140 is described here as including a plurality of magnets 152, the reader 140 can include one magnet, if it desirous to do so. In one embodiment, the magnets 152 (or magnet) are arranged to meet the securing specifications, including, without limitation, a latching force, a lifting force, size, reader orientation, combinations thereof, or the like. For example, the latching force is the force that secures the magnets 152 of the flexible seal 144 to the metal ring 148. The lifting force is the force required to separate the magnets 152 and the metal ring 148. The latching force is a function of at least one attraction factor, which includes the size of the metal ring 148 (e.g., the diameter or side-to-side measurement), the thickness of the metal ring 148, the size of the magnets 152, the quantity of magnets 152, the spacing of multiple magnets 152, and the strength of the magnets 152. The attraction factors can increase or decrease the magnetic attraction between the flexible seal 144 and the metal ring 148, thereby increasing or decreasing the latching and lifting forces. For example, the attractive force between a magnet and a thicker piece of metal is greater than the attractive force between the magnet and a thinner piece of metal. As another example, a stronger magnet has a greater attractive force than a weaker magnet.

The flexible seal 144 and the metal ring 148 maintain the same latching force regardless of the size or orientation of the reader 140 to meet the water- and dust-resistance requirements. At least one of the attraction factors can be adjusted to maintain the same latching force based on reader size and/or orientation. For example, a smaller reader can have a thinner metal ring and a stronger magnet. The thinner metal ring results in a weaker attraction. Therefore, the stronger magnet is required to maintain the same latching force as a reader with a thicker metal ring. Furthermore, the lid 142 closes due to gravity to prevent the lid 142 from remaining open or being propped open. For example, a spring, a cam hinge, a pivot hinge, a strut hinge (e.g., gas strut hinge), or the like can be incorporated to close the lid 142 under gravity.

The flexible seal 144 can be composed of a ductile, flexible, and/or elastic material, including, without limitation, a polymer, plastic, silicone, rubber, nylon, polyester, microfiber, or combinations thereof. The flexible seal 144 can also be composed of materials which are capable of being hinged, thereby allowing for an accordion-style movement, including, without limitation, metal, hardened plastic, the like, or combinations thereof. For example, the flexible seal 144 including a first rectangular piece having a thickness and two long sides adjoined two short ends and a second rectangular piece having a thickness and two long sides adjoined two short ends. The first and second pieces via a hinge along one their respective long sides. When the flexible seal 144 is fully extended, the long sides of the first and second pieces sharing the hinge are proximal to each other or contact each. When the flexible seal 144 is fully collapsed, the first and second pieces stack on top of each other, thereby effectively doubling the thickness. Though the flexible seal 144 is discussed as being accordion-style with two pieces, the flexible seal 144 can include at least 2, 3, 4, 5, 6, 7, 8, 9, 10, 15, 20, 25, 30, 35, 40, 45, 50, 75, 80, 90, or 100 pieces.

The flexible seal 144 is water- and dust-resistant, thereby helping keep water and dust out of an inner compartment 146 of the reader 140. The internal compartment 146 includes the cartridge receptacle 114.

The reader 140 also includes the housing 112 and the display 116, such as a screen, such as a touchscreen, lights, or other visual indicators. The touchscreen used to display information, such as analysis results, to the user can also be used by a user to input to the reader 140. An audible output can include a speaker, buzzer, or other audible indicators. The output, visual and/or audible, can be output through an external device, such as a computer, speaker, or mobile device connected physically or wirelessly to the reader 140. The display 116 can include tactile prompts, input, and output. The output, whether visual, audible, or tactile, can output data, including the collected analysis data and interpretative data indicative of diagnosis, including the presence or absence of an infection, disease, or condition within the patient or the patient sample. An example can include the presence of hemozoin within the patient sample. The interpretive data output can be based on the analysis data collected and processed by processing circuitry of the reader 140.

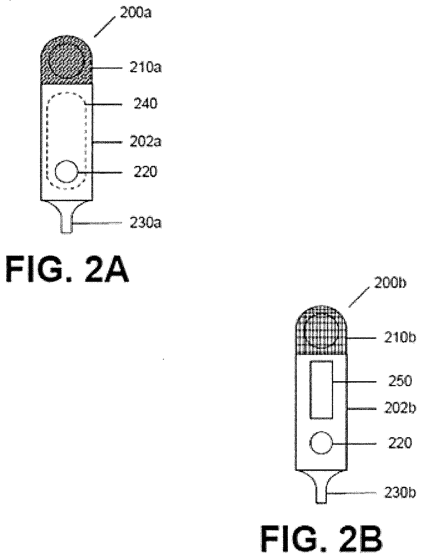

FIGS. 2A-2B illustrate example cartridges 200a and 200b. Each of the example cartridges 200a, 200b include a housing 202a, 202b an upper portion 210a, 210b and a lower portion 230a, 230b. The cartridges 200a, 200b can include a sample chamber, such as 240 of FIG. 2A, that is internal to the cartridge 200a, 200b and can store a patient sample, such as a blood sample, within the cartridge 200a, 200b. The cartridges 200a, 200b transport or store a patient sample for analysis, or reading, by a reader. Further, the cartridges 200a, 200b can interface with the reader to assist with or facilitate the reading or analysis of the patient sample stored within the cartridge 200a, 200b. That is, the cartridge 200a, 200b can include features, such as an optical window 220 and an electrophoresis element 250 of cartridge 200b of FIG. 2B to assist with the analysis of the patient sample within the cartridge 200a, 200b. The cartridge 200a, 200b can also transfer all or a portion of the patient sample to the reader for analysis of the patient sample. The patient sample, or portion thereof, from the cartridge 200a, 200b can be transferred to a blood sample chamber of the reader or to another location of the reader, or external from the reader, for analysis of the patient blood sample.

The cartridges 200a, 200b can be condition, disease or ailment specific or multiple condition, disease or ailment specific. The cartridges 200a, 200b can include various features, external or internal, that customize a particular cartridge for the analysis of a specific, singular or multiple, condition, disease and/or ailment. The cartridge can include the patient sample size volume of the cartridge, various diluents, stains (e.g., fluorescent, chromogenic, or the like) and/or reagents in the cartridge, the interface of the cartridge with the reader and other design and/or construction specification of the cartridge in relation to one or more particular conditions, diseases and/or ailments.

The housing 202a, 202b of the cartridge 200a, 200b can include structural, material and/or geometric features that assist or facilitate the analysis and/or acquisition of the patient sample. Such features can include internal chambers, such as the sample chamber 240 of FIG. 2A, to store the patient sample or other fluids or compounds, that are sized to ensure adequate sample size for the analysis of the collected patient blood sample, interfaces that interact with, engage, or facilitate the systems of the reader during analysis of the patient sample. Other features can include environmental controls that maintain the collected patient sample in a suitable condition for analysis, and other features and/or considerations. For example, an internal chamber of the reader could manually or automatically interface with the inserted cartridge via a port to cause diluents, stains (e.g., fluorescent, chromogenic, or the like) and/or other chemical treatments to mix with the patient sample in the cartridge. Such a port would be a passage, like a tube, that connects the sample chamber of the cartridge with the port so fluids can be added to the cartridge. The addition of such external fluids can be triggered automatically or manually when a user actuates a switch or other actuator, which the user may do in response to a user prompt to do so. The cartridge housing 202a, 202b can be formed of a suitable material such as a plastic, composite and/or metal to create a robust, disposable cartridge 200a, 200b. Additionally, the housing 202a, 202b material can be selected for the ability to be sterilized, such as sterilizing the cartridge 200a, 200b prior to use, for reuse or for killing pathogens prior to disposal.

Environmental considerations can also be used in the determination of a suitable material(s) for the cartridge housing 202a, 202b. Such environmental considerations can include the biodegradability of the housing material, the recyclability of the housing material, the incineration by-products of the housing material and other environmental considerations. These environmental considerations can reduce the environmental impact of the disposal, recycling and/or reuse of the cartridges 200a, 200b after use.

The housing 202a, 202b of the cartridge 200a, 200b can include a patient identification marker or an area to apply or mark patient identification onto the cartridge 200a, 200b. This marker could be in machine readable or human readable form or both. The patient identification allows the correlation of the analysis of the collected patient sample with a particular patient. Additionally, the reader can detect the patient identification marker to correlate the analysis with a patient, including automatically appending the analysis results to a patient's medical records. In an example embodiment, the patient identification can be obfuscated to remove patient personal information, such as a name, from the cartridge 200a, 200b, instead the patient can be assigned a random number, or sequence of characters, that is correlated to the particular patient in the reader, a computer or other system. The marker can also contain information on what test(s) to run and what to do with any results collected.

Patient diagnostic and demographic information can also be used for analysis to determine trends or emergence of conditions, disorders, diseases and/or ailments. This analysis can be used to prevent or minimize the spread of the condition/disorder and/or targeted diagnosis and/or treatment of the condition/disorder. For various conditions once properly diagnosed, such as a sickle cell and other hemoglobin conditions, geographical correlation of the prevalence of the condition can be used to perform measures to mitigate and minimize the effects of the condition on the target population.

The upper portion 210a, 210b of the cartridge 200a, 200b can include identification marker(s), such as a color, pattern, name, or other distinguishing features. The identification marker can be used to indicate the use of the cartridge 200a, 200b for the analysis of a specific condition(s), disease(s), and/or ailment(s). This can provide a clear, visual indication to a user that the cartridge 200a, 200b is to be used with specific analysis or analyses.

Additionally, the upper portion 210a, 210b can be a portion of a sample collection element, such as a suction bulb, actuation element, or capillary tube to assist or facilitate the collection of the patient sample into the cartridge 200a, 200b. As a suction bulb, the upper portion can be formed of a resilient or flexible material capable of deforming in volume to assist in the uptake of a patient sample within the cartridge 200a, 200b. As an actuation element, the application of pressure or other input by a user, other or device to the upper portion 210a, 210b of the cartridge 200a, 200b can actuate the passive or active acquisition of a patient sample into the cartridge 200a, 200b in preparation for analysis, such as extending and/or retracting a needle or capillary tube. A capillary tube or plane is one means of passively collecting the sample with no user or machine pressure required.

Further, the upper portion 210a, 210b can contain a diluent, marker, reagent or other fluid or substance that is stored internally in a chamber and that can be released into and/or mixed with the patient sample within the cartridge 200a, 200b. Application of pressure to the upper portion 210a, 210b of the cartridge 200a, 200b can introduce the contained substance or fluid into the patient sample within the cartridge 200a, 200b which mixes the patient sample with the contained substance(s) or fluid(s). Example diluent ratios can include from 1:0.5 to 1:100. The contained substance or fluid can assist with the analysis of the patient sample, preparation of the patient sample for analysis, preservation of the sample for analysis or other desirable or necessary patient sample modification for efficient and effective analysis of the patient sample.

Additionally, the upper portion 210a, 210b of the cartridge 200a, 200b can be contoured and/or shaped to provide a comfortable, ergonomic, and/or easy grip for a user to handle the cartridge 200a, 200b during insertion and/or extraction into/from the reader or diagnostic device. Alternatively, the surface texture of the upper portion 210a, 210b can be such that it improves the ability of a user to grip the cartridge 200a, 200b.

The optical window 220 can be included on the cartridge 200a, 200b, which allows light to pass into and/or through a portion of the cartridge 200a, 200b such as a sample chamber containing the patient sample, such as 240 of FIG. 2A. The ability to pass light into and/or through the sample volume within the cartridge 200a, 200b can be a necessary step during analysis of the patient sample within the cartridge 200a, 200b. The optical window 220 can be a material and/or construction that necessarily or desirably alters light entering the optical window 220 as a part of the analysis of the patient sample within, such as collimating, filtering, and/or polarizing the light that passes through the optical window 220. Alternatively, the optical window 220 can be transparent or translucent, or can be an opening within the housing 202a, 202b of the cartridge 200a, 200b. The cartridge 200a, 200b can include a reflector opposite the optical window 220, 220b that reflects the incoming light back through the optical window 220a, 220b or through another optical window, or can include a further optical window opposite the light entry window to allow light to pass through the cartridge 200a, 200b.

An electrophoresis element, such as 250 of cartridge 200b of FIG. 2B, can assist with performing an electrophoresis analysis of a patient sample within the cartridge 250. The electrophoresis element 250 can include electrodes to establish an electrical gradient across the element to perform the electrophoresis analysis. A cover can be included to protect the electrophoresis element, while still allowing the results to be viewed either through the cover or by removal of the cover. The cover can be optically transparent to allow optical viewing of the results and/or the electrophoresis process being performed. Light can be reflected off of and/or transmitted through the electrophoresis element 250 to assist with viewing the results displayed thereon. The cartridge 200b can include one or more structural features to facilitate the transmission of light through the electrophoresis element 250.

The lower portion 230a, 230b can house or be a portion of the sample collection system. In the examples shown in FIGS. 2A and 2B, the lower portion 230a, 230b can include a channel or tube through which the patient sample can be transferred into the interior of the cartridge 200a, 200b. The lower portion 230a, 230b can also house a portion of the sample collection system, such as an extendable needle like a lancet or a capillary tube through which the patient sample can be transferred to the interior of the cartridge 200a, 200b.

The lower portion 230a, 230b can also include elements and/or systems to assist with the analyzing and/or storage of the patient sample. This can include an interface and/or mechanism to release at least a portion of the patient sample from within the cartridge 200a, 200b into the reader and/or a barrier or seal that restrains and/or preserves the patient sample within the cartridge 200a, 200b.

The lower portion 230a, 230b can further include an indicator that is visible once the cartridge 200a, 200b has been previously used. This can prevent cross-contamination of patient specimens and/or prevent the reuse of a single-use cartridge 200a, 200b which could alter or otherwise compromise the results of the patient sample analysis. The indication can be structural in nature, with an alteration, such as a removal or break in a portion of the cartridge 200a, 200b housing 202a, 202b of the lower portion 230a, 230b that is a visible once the cartridge 200a, 200b has been used or has acquired a patient sample. Additionally, the lower portion 230a, 230b can deform after acquisition of a patient sample within the cartridge 200a, 200b, which prevents further collection of a patient sample(s) using the cartridge 200a, 200b. The indication could be electrical.

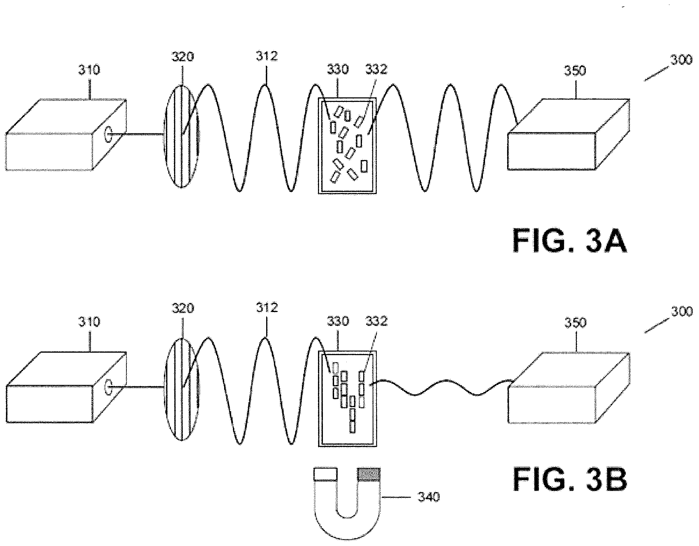

FIGS. 3A-3B illustrate an example magneto-optical detection (MOD) system 300. The MOD system 300 includes a light source 310 that emits light 312, a polarizer 320, a patient sample 330, a magnet 340 and a photodetector 350. Some diseases and conditions result in the release of or changes in a magnetic, or paramagnetic, component of a patient's sample. An example patient sample can include blood which includes hemozoin that contains iron-a magnetic compound, the amount and/or concentration of which can be used to determine the presence and/or intensity of a condition or disease, such as malaria. The transmission of light 312 through a patient sample 330 in a varying magnetic field can be used to detect the presence of and determine, absolute or relative, concentrations of magnetic and non-magnetic components within the patient sample 330.

FIG. 3A illustrates the transmission of light 312, from the light source 310, through the patient sample 330 in a magnetic field in a first state, such as a low strength magnetic field. In this example, a magnetic component 332 of the patient sample 330 is randomly arranged allowing for a measurable transmittance of light 312 through the patient sample 330. The transmitted light 312 is received by the photodetector 350 and characterized, such as by properties including frequency, intensity, distribution, wavelength or other light properties or characteristics. This first light value is a base value that can be used to measure the relative change in at least a property or characteristic of the light transmitted through the patient sample 330 with an alternate, varying or changeable magnetic field applied.

FIG. 3B illustrates the transmission of light 312, from the same light source 310, through the patient sample 330 in the presence of an applied magnetic field in a second state, such as a higher strength magnetic field than the first state. The strength of the magnetic field applied to the patient sample 330 can be increased from the first state to the higher strength second state by altering the proximity of a magnet(s) 340. The application of a higher strength magnetic field causes the ordered alignment or arrangement of the magnetic, or paramagnetic, components 332 of the patient sample 330. This ordering or alignment effects the transmission of light 312 through the patient sample 330, which is a second value that can be detected by the photodetector 350. An effect can include the reduction or increase of light 312 transmitted through the patient sample 330. The comparison or measurement of the first light value in a magnetic field in a first, lower, state, and the second light value in a magnetic field in a second, higher, state, can be used to determine the presence of a disease or condition or the intensity of the disease or condition, such as the level of infection.

An example disease detectable by a MOD system, such as that of FIGS. 3A-3B, can include malaria. Malaria can be caused by a variety of different plasmodium parasites which infect the hemoglobin containing red blood cells of a host. The plasmodium replicate within the red blood cells, eventually destroying the red blood cells. The plasmodium parasite(s) release hemozoin as a byproduct after having ingested an infected patient's healthy hemoglobin. Hemozoin in a patient's blood is a biomarker of malaria. Hemozoin is a paramagnetic compound and is thus sensitive to magnetic fields. Hemozoin within a patient sample can be detected by a MOD system due to a differential light transmission characteristic(s) in different magnetic fields. The differential light transmission characteristic can be indicative of several infection characteristics, such as the presence of the parasite, the parasite infection levels, the parasite species, and other infection characteristics.

A MOD system, such as the example of FIGS. 3A-3B, can be integrated into a reader, such as the example shown in FIGS. 1A-1B, or can be external to a reader. In the example in which the MOD system is external to a reader, the MOD system requires the ability to pass light through the patient sample, within a cartridge or reader, and detect properties or characteristics of the light transmitted through the patient sample in a varying/changeable magnetic field. Alternatively, the MOD system and components can be split between the reader and external to the reader, with a portion of the MOD system and components located internal to the reader and another portion of the MOD system and components located external to the reader.

Additionally, an example MOD system can include only the optical component. Light from a light source is transmitted through a patient sample and the transmitted light is received by a light detector that can determine and measure properties/characteristics of the transmitted light or can transmit information from which the properties/characteristics of the transmitted light can be determined or measured. In this example, the magnetic component of the MOD system is either absent from the system or is not used during analysis of a patient sample. Instead, the patient sample can be analyzed based on the light transmission differential or characteristics of the transmitted light.

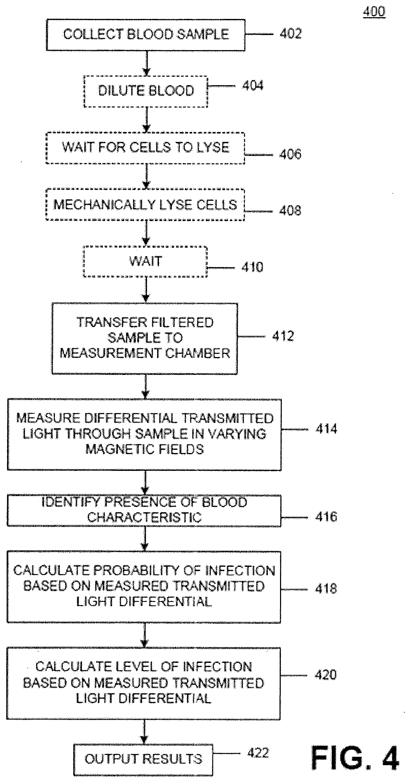

FIG. 4 is an example analysis method 400 using a MOD system, such as the one shown in FIGS. 3A-3B. The analysis of a patient sample, which is patient blood in this example, is performed to determine a blood characteristic, which can include the presence of a disease or condition, quantification of a disease or condition, likelihood of the presence of a disease or condition, a characteristic that can be indicative of a disease or condition, a quantification of a characteristic that can be indicative of a disease or condition, or other blood characteristic that can be effected by the presence of a disease or condition of the patient. The example method of FIG. 4 is performed using a reader and cartridge system, such as the example shown in FIGS. 1A-1B, and the MOD system is included within the reader which can include additional systems or elements to analyze, quantify, identify or otherwise determine characteristics of a patient sample that can be indicative of the presence of a disease or condition of the patient.

An initial step 402 of the method 400 can include the collection of a patient sample for analysis, in this example, a blood sample. Alternative or further patient samples, such as saliva, tissue or other bodily fluids can be collected for analysis by one or more systems of the reader.

The collected blood sample 402 can then be prepared, if necessary or desired, for analysis. The preparation of the blood sample can include diluting the blood 404, which can be done by mixing the collected blood sample with a diluent, such as deionized water or other fluid that dilutes the blood sample. The diluent can alter the viscosity of the blood sample, the opacity or translucence of the blood sample, or otherwise prepare the blood sample for analysis using the reader. Preferably, the diluent does not impact the resulting analysis of the blood sample and assists with preparing the blood sample for analysis. This can include lysing the cells of the blood sample to release the various cellular components for analysis, such as detection or quantification, by the reader. Lysing agents can include fluids, such as water or various chemicals, and powders.

Cellular lysing can take time, so a requisite amount of time may be required 406 to ensure adequate cellular lysing has occurred within the blood sample in preparation for analysis. The lysing of the cells of the blood sample can occur naturally, as part of a cellular death or destruction cycle, or can be enhanced or performed using chemical or mechanical lysing techniques 408. As previously discussed, adequate time can be waited 410, such as 5 minutes for lysing in water and 15 seconds for mechanical lysing using sonication, to achieve adequate lysing of the cells of the blood sample in preparation for analysis of the sample using the reader.

The blood sample can then be filtered before being transferred into a measurement chamber 412 of the cartridge. Filtering the sample can remove one or more components of the sample, such as debris from cell lysing, clots or agglomerations of cells, or other components that could affect the analysis or are otherwise undesirable or unneeded in the sample to be analyzed. An alternative approach is to filter, so the hemozoin for example, are one of the components left. The filter can be an element having structural features, such as pore size, or chemical features that allow the filter to restrain, remove, attract, or otherwise filter a particular component from the patient sample. An example filter can have a pore size of 1-5 microns to filter a blood-type or other patient sample. The patient sample or blood can be passed passively, by Brownian motion, or actively, by a pressure differential, through or across a fixed filter to remove the particular component. Alternatively, the filter can move through, about or be placed in the patient or blood sample to filter a component(s) from the sample. Passing through the filter can also cause or enhance cell lysing.

A further preparation of the patient blood sample can include cleaning and/or concentrating the patient sample prior to analysis. Cleaning or concentrating the patient sample can include removing unwanted components of the patient blood sample prior to analysis. The various unwanted components are typically dispersed throughout the patient's blood and can interfere with an accurate reading of the patient sample. For example, the unwanted components can add noise to the detected data signal, can move in and out of the light transmission path that is transmitted through the sample, or can otherwise obstruct the analysis of the patient sample.

An example cleaning or concentrating the patient sample can include appropriately diluting the patient sample anywhere between 100:1 to 2:1 or any other desired amount. The diluted sample lowers the effective concentration of the compound(s) being studied. The sample is then lysed, such as by sonication. The lysed patient sample is then centrifuged to separate one or more desired components of the patient sample from the remaining portion of the patient sample. The remaining portion of the patient sample, the supernate of the centrifuged sample, can be disposed of so that the one or more desired components of the patient sample remain or the supernate might be the desired component. During centrifuging, hemozoin forms small pellets while some other blood components remain in suspension to form the supernate. The concentrated portion, in this specific example the hemozoin, is then diluted to a desired end volume. The re-diluted hemozoin is sonicated to loosen the hemozoin from the walls of the centrifuge chamber where it tends to adhere during centrifugation. Analysis of the cleaned or concentrated sample can then be performed using one or more systems of the reader.

Another example of cleaning or concentrating the patient sample prior to analysis can include passing the lysed diluted patient sample over a magnetic surface, such as a column of ferrite balls in a magnetic field. The magnetized ferrite surface attracts the magnetic components of the patient sample, while the remainder of the sample passes through which concentrates the magnetic component(s) of the patient sample and separating, or cleaning, the magnetic component(s) from the remainder of the patient sample. The magnetic component(s) of the patient sample can then be washed from the ferrite surface after removing the magnetic field. The sample can then be diluted, which can also be performed by the washing of the magnetic components from the ferrite surface, to an appropriate or necessary volume for analysis. The ferrite surface can also be sonicated or vibrated to assist with removal or washing of the magnetic component of the patient sample from the ferrite surface.

In further embodiments, the measurement chamber can be a chamber within the reader, with the patient blood sample transferred to the measurement chamber of the reader from the cartridge. An interface of the reader or cartridge can transfer a portion of the patient or blood sample from the cartridge into the measurement chamber of the reader. The patient sample can be transferred from the cartridge to the reader using a pressure differential, such as negative pressure in the reader measurement chamber to draw the sample from the cartridge or positive pressure applied to the cartridge to push the patient sample from the cartridge into the reader measurement chamber. The sample can be transferred from the cartridge to the reader through the same opening as the patient sample was originally transferred into the cartridge or through another opening or conduit of the cartridge or the chamber of the cartridge within which the patient sample is contained. Alternatively, the reader can include a piercing element to pierce a portion of the cartridge to withdraw the patient sample, or a portion, from the cartridge.

Light is then transmitted or passed through the blood sample and measured in a varying magnetic field 414, such as the system of FIGS. 3A-3B. The application of a varying magnetic field to the blood sample can cause magnetic, or paramagnetic, components of the patient, or blood, sample to align with the polarity of the applied magnetic field. The alignment of the magnetic, or paramagnetic, components of the blood sample affects the transmission of light through the blood sample. As such, a differential of light transmittance through the blood sample can be established by transmitting light through the blood sample with the application of a magnetic field in a first state, such as a lower strength or intensity of magnetic field or the absence of a magnetic field, and then applying the same light, same intensity and wavelength, through the blood sample while the magnetic field is applied in a second state, such as a higher strength or intensity of magnetic field or the application of a magnetic field. The differential of the transmitted light through the blood sample in the two states can indicate the presence and amount of a paramagnetic compound(s) within the blood sample. The applied magnetic field can be from one or more permanent magnets or electromagnet(s) that can be energized. Either the blood sample or the magnets can be moveable and in some examples, either is moveable or both are moveable. The magnetic field applied to the blood sample can be attuned to preferentially affect a specific magnetic, or paramagnetic, component(s) of the patient or blood sample. The application or variance of the magnetic field can also affect or impact other portions or components of the patient or blood sample.

The measured light transmission differential can be used to identify the presence of a sample, or blood, characteristic 416 as indicated by a magnetic or paramagnetic component of the analyzed blood sample. The blood characteristic can include the release or breakdown of hemoglobin, or components or products thereof, which can be indicative of a disorder or disease.

Based on the measured light transmission differential, a probability of an infection can be determined 418. The probability of an infection or disease can be expressed as a numerical value or a subjective likelihood, such as a high or low probability, based on comparing the measured light transmission differential to a database of known correlated measured values and infection probabilities, an algorithm to correlate measured light transmission differential with infection probability, a statistical analysis of the measured light transmission differential to determine an infection probability, or repeated analysis of the sample to determine an infection probability based on the measured light transmission differential. Additional statistical techniques, algorithms or other analysis techniques can be applied using the measured light transmission differential to determine an infection probability based on the collected and analyzed blood sample.

In addition to calculating an infection probability, a level of infection can be determined based on the measured light transmission differential 420. As with the calculation of the probability of infection, various statistical techniques, algorithms, or other analysis techniques can be applied to determine a level of infection. Additionally, a database, remote or local to the reader, can be used in the calculation of the level of infection. The database can contain algorithms, historical data, correlations of infection level to measured light transmission differentials, or other data that can be used to calculate the level of infection. Additionally, the sample can be analyzed multiple times to confirm or generate additional data to be used for the calculation of the level of infection or the probability of infection.

Once the analysis of the blood sample is complete, the results can be output 422. The output of the results can include the identified blood characteristic(s), which can include a disease, condition or ailment, the calculated probability of infection, the calculated level of infection, the calculated level of the bio-marker being measured and other information relevant to or determined, calculated, or identified during the analysis of the blood sample. The output can be displayed or relayed to the user in a visual output, such as on a display, auditory, such as by a speaker, or other manner. This can include transmitting the output results to an external device, such as a computer, through a wired or wireless connection or communication protocol.

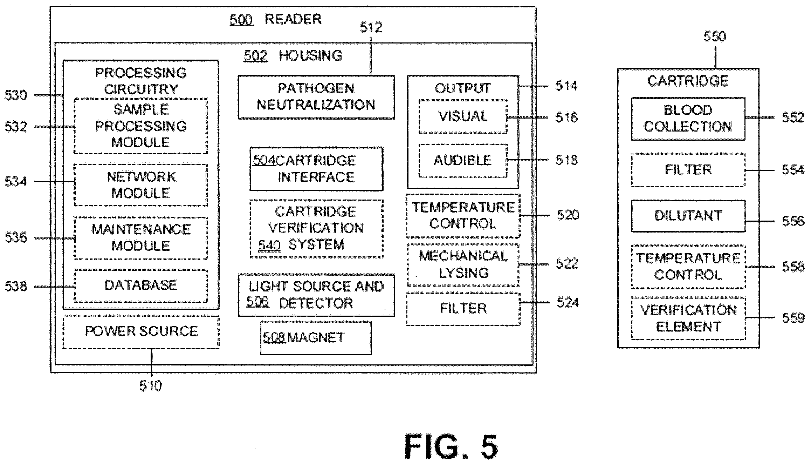

FIG. 5 illustrates an example reader 500 and a cartridge 550. The reader 500 can include all or a portion of the required systems or elements required to perform analysis of a patient sample. The cartridge 550 can include none or a portion of the systems or elements required to perform analysis of the patient sample. The reader 500 and cartridge 550 interface to perform the analysis, such as the method 400 of FIG. 4, of a patient sample.

The reader 500 includes a housing 502 that surrounds and encloses some portion or all of the reader components. FIG. 5 shows that the housing encloses all components of the reader 500, however, one of skill in the art will appreciate that any one or more components can be external to the housing, as needed or desired. As previously discussed, the housing 502 of the reader 500 is constructed of suitable materials in a suitably robust construction such that the reader 500 is rugged and portable. Example materials that can be included in the housing 502 include plastics, metals, and composites. The housing 502 can be constructed of multiple or a singular material and can include geometry or structural features that enhance the usability of the reader 500. Such features can include a smooth outer surface that is easily cleaned, grips or handles for carrying the reader 500, shock protection or increased structural strength in locations to prevent damage to the internal components of the reader 500, insulation to limit the transfer of heat through the housing 502 or shield magnetic fields sourced within the housing 502, a membrane or construction to prevent the intrusion of moisture and dust into the interior of the reader 502, connections, ports or interfaces for connecting the reader 500 to an external element or device using a physical or wireless connection, instructions regarding the use of the reader 500, identification markings such as a serial number or additional necessary or desirable features that can facilitate the safe, effective, efficient or proper use of the reader 500. The housing 502 can feature access points, such as removable or openable panels, to allow access to the interior of the reader 500 for maintenance or repair of the internal components, elements or systems of the reader 500. Additionally, the housing 502 of the reader 500 can be removable or separable from the other components, elements or systems of the reader 500, allowing the replacement of the housing 502, easing the cleaning of the housing 502, providing access to the components, elements or systems of the reader 500 or other abilities that require or are made easier by the removal of the housing 502 of the reader 500.

The portability of the reader 500 can be an important consideration in the design and packaging of the reader 500, including the housing 502. The reader 500 may need to be rugged and easily transported so that it can be moved to and used in a variety of embodiments. Considerations, such as operating environment and access to infrastructure, can be considered when designing or constructing the reader 500 such that the reader can be used safely, effectively, and efficiently in a variety of environments or locations reliably. Depending on the environment of and infrastructure available in a particular location in which the system is to be used, the housing can be customized to best operate in that location by the addition or modification of existing reader features. Alternatively, the reader 500 can be designed or packaged to be more permanently located, such as in a laboratory, clinic, or other setting.

The housing 502 of the reader 500 includes a cartridge interface 504 that interacts with or engages the cartridge 550 for analysis of a patient sample. The cartridge interface 504 can be a slot that is shaped to receive the cartridge 550. The user inserts the cartridge 550 into the slot in preparation for analysis of the patient sample. The slot can include internal geometry that aligns or orients the inserted cartridge 550 in a proper alignment or orientation for the components, elements or systems of the reader 500 to perform the requisite or desired analysis of the patient sample contained within the cartridge 500. For example, the cartridge interface 504 can accept a variety of cartridges 550 having different cross-sections, such as square, rectangular, and circular cross-sections. The unique shape of each cartridge 550, the unique cross-section, can interact with the geometry of the cartridge interface 504 to properly align the cartridge 550 within the reader 500 for analysis. For example, the circular cross-section cartridge can insert into the cartridge interface 504 to a first position at a first orientation, the square cross-section cartridge can insert into the cartridge interface 504 to a second position at a second orientation. The various orientations and positions a specific cartridge 550 can be inserted into the cartridge interface 504 can be the same or different for multiple disease-specific cartridges 550.

The reader 500 can also include a cartridge verification system 540. The cartridge verification system 540 can be integrated with or separate from the cartridge interface 504 or included internal to or external from the reader 500. The cartridge verification system 540 can verify the legitimacy of a cartridge to assist with efficient and effective analysis of a patient sample. An example verification system 540 can include a verification element 559 of the cartridge 550 that interacts with the cartridge verification system 540 to verify the cartridge prior to further processing of the patient sample. Once the cartridge is verified, further analysis of a patient sample contained within the cartridge can be allowed to proceed. The verification of the cartridge can be the threshold analysis of the in vitro diagnostics process of the patient sample, in some examples. This verification can include limiting the analysis to a specific single or multiple analyses based on the cartridge verification.

A positive engagement or lock in the reader 500 can engage the cartridge 550 when properly and fully inserted. This engagement can also provide a tactile, audible, or visual cue to the user to signify proper insertion or interfacing of the cartridge 550 and reader 500. An example positive engagement or lock can include a notch and protrusion arrangement, the notch is sized to receive and releasably restrain the protrusion when engaged such that the notch of one element, the reader 500 or cartridge 550, engages the protrusion on the opposite element, reader 500 or cartridge 550, to releasably connect, interface with or engage the two elements, the reader 500 and cartridge 550, together. When prompted, such as when the analysis is completed or an error situation, the user can remove the cartridge 550 from the reader 500.

The cartridge interface 504 can also include an actuator or other element of the reader 500 that assists with the proper insertion or interfacing of the cartridge 550 and reader 500. The actuator can engage the cartridge 550 before the cartridge is fully inserted, the actuator can then position the cartridge 550 in a proper alignment or orientation with the reader 500 for the reader 500 to analyze the patient sample within the cartridge 550. When prompted, such as automatically by the reader 500 or manually by the user, the actuator can "eject" or disengage the cartridge 550 from the reader 500. The disengagement can fully or partially remove the cartridge 550 from the reader 500. Alternatively, the actuator can assist with the engagement or interfacing of the cartridge 550 with the reader 500 and not with the disengagement of the cartridge 550 and reader 500. In this example, the user can be required to remove the cartridge 550 from the reader 500 when prompted.

The cartridge interface 504 can be shaped to engage one or more specific cartridges 550, which prevents the insertion of an incorrect or improper cartridge 550 within the reader 500. The cartridge interface 504 can also be reconfigurable, either manually by a user or automatically by the reader 500 to accommodate a specific cartridge design to perform one or more specific analyses of a patient sample. For example, a user can input a desired or required analysis to be performed on a patient sample, the reader 500 can then reconfigure or prompt the reconfiguration of the cartridge interface 504 to accept a specific cartridge 550 that corresponds to the requested analysis.

For example, the cartridge interface 504 can include multiple configurable elements, such as panels, that can be configured or arranged automatically in response to a received analysis to be performed, such as a user-selected infection or disease for which to analyze the patient sample. The now configured or arranged configurable elements of the cartridge interface 504 are in a specific geometry into which only a compatible cartridge can be inserted. The analysis to be performed can be an input by a user into the reader 500 or from a remote administrator or system. In a further example, a specific cartridge interface 504 can include removable or replaceable cartridge interfaces 504 that can be removed from or inserted in the reader 500. Each cartridge interface can include geometry to accept a specific cartridge design(s). Additionally, the inserted cartridge interface 504 can be detected or otherwise communicated to the reader 500 and the reader 500 can limit available options, such as the analyses that can be performed, based on the inserted cartridge interface 504. Each cartridge interface 504, or cartridge interface 504 design or geometry, can correspond to a specific analysis or analyses. Further, the reader 500 can be limited to the specific analysis or analyses corresponding to the particular cartridge interface 504 or cartridge interface 504 geometry.

In a further example, the cartridge interface 504 can initially accept any inserted cartridge. Once a cartridge is inserted, the cartridge interface 504, a sensor or other reader 500 system or element can detect the cartridge type and the corresponding analysis or analyses that can be performed based on the cartridge type. The cartridge interface 504 can manipulate the cartridge position or orientation, the reader 500 can properly position or orient analysis systems or elements relative to the cartridge, or the cartridge interface 504 or reader 500 systems or elements perform the analysis or analyses corresponding to the cartridge type.

Also, a sample processing module 532 of the processing circuitry 530 of the reader 500, or an external sample processing system or element, can alter the processing of the sample analysis data to correct, compensate or otherwise modify the collected sample analysis data based on the type of cartridge inserted within the reader 500. Instead of or in addition to positioning or aligning the cartridge or reader 500 analysis systems relative to the reader, the processing of the collected sample analysis data can be manipulated or modified to compensate based on the type of cartridge inserted. Additional modifiers can include compensating for position/alignment errors caused by improper alignment/positioning of the cartridge relative to the analysis systems or elements.

Further, the cartridge interface 504 can include multiple orientation or alignment features that engage specific cartridge 550 features to properly align a specific, inserted cartridge with a specific analysis process. For example, a first cartridge for a first specific analysis is inserted into the cartridge interface 504 which guides, aligns, or orients the first cartridge properly in a first position for the first analysis to be performed, a second cartridge for a second specific analysis can be interested in the same cartridge interface 504, which can properly guide, align, or orient the second cartridge in a second position for the second analysis to be performed. In this manner, the cartridge interface 504 ensures the proper positioning of a variety of specific cartridge designs within the reader 500 allowing a corresponding variety of specific analyses to be performed, each analysis corresponding to one or more specific cartridge designs.

The cartridge interface 504 can also include a number for position points corresponding to various steps of analysis. For example, an analysis can require that the cartridge 550 is inserted partially to a first position within the reader 500 to perform a first step of the analysis, the reader 500 can prompt the user to advance or move the cartridge 550 to a second position, such as further insertion of the cartridge 550 within the reader 500, to perform a further step of the analysis. Each position can include a tactile, audible, or visual indication to a user manually inserting the cartridge 550 within the cartridge interface 504 to assist the user with properly position the cartridge 550 within the cartridge interface 504. An actuator, such as described previously, can position the cartridge 550 at the various analysis require positions automatically, or can assist the user with the cartridge 550 positioning.

Insertion of the cartridge 550 into cartridge interface 504 of the reader 500 can automatically initiate or prompt a user to initiate analysis of the patient sample contained within the cartridge 550. An actuator or sensor can be connected to the processing circuitry of the reader 500 and triggered by or sense the insertion of the cartridge 550 to automatically initiate or to prompt a user to initiate the analysis of the patient sample. Initiating analysis of the patient sample can include powering-up, preparing, and running the various analyses systems or devices, such as a light source and detector 506 or mechanical lysing 522. In some examples, the user need only insert the cartridge 550 in the reader 500 to actuate or trigger the entire diagnostics process to an output.

The cartridge interface 504 and additional elements, such as guides or actuators can be integrated into the housing 502 of the reader 500 or can be separate components, elements or systems. Each of the additional elements can be further separable from each other allowing for replacement, substitution, repair or maintenance of the additional elements as necessary or required.

The reader 500 can include a single cartridge interface 504, such as the example shown in FIGS. 1A-1B, or can include multiple cartridge interfaces 504 in the same reader 500. The multiple cartridge interfaces 504 can allow the reader 500 to analyze multiple patient samples simultaneously or in succession by allowing more than one cartridge 550 to be interfaced with the reader 500. Additionally, each of the multiple cartridge interfaces 504 can accept the same or different cartridges to perform the same or different analyses. Further, in conjunction with a multi- or singular cartridge interface 504, a guide, rack, carousel or system can hold multiple cartridges in preparation for analysis. The guide, rack, carousel or system can feed or guide, actively or passively, cartridges 550 to the reader 500 by the cartridge interface 504 allowing multiple patient samples and/or cartridges 550 to be analyzed with minimal interruption between the analyses.

The reader 500 shown in FIG. 5 includes a light source and detector 506. The light source and detector 506 can be part of a MOD, the optical portion, or other analysis and/or detection system within the reader 500, to be used in performing analysis of patient samples. The light source emits light and the light detector receives light, signals, or outputs from the light detector. The detected light can be used to quantify /or characterize the light received by the light detector. In example embodiments, the light source and detector can be arranged opposite one another, separated by a distance along a single axis. In this example, the light detector can receive light emitted from the light source across the distance, which can include an intervening object such as a patient sample. In this example, the laser and detector are positioned on opposing sides across the patient sample contained in the cartridge and the cartridge has an optical window(s) that allow for complete transmission of the laser light through the patient sample. The laser light transmission path through the patient sample can be entirely through the fluid, below any free surface of the fluid if the sample chamber is not completely full of the patient sample. Alternatively, the light source and light detector can be arranged offset from one another allowing the light detector to quantify or characterize light reflected or refracted by an object, such as a patient sample. Further, multiple light sources or light detectors can be included in the reader 500.

The positioning and structure of the cartridge 550 within the reader 500 can be such that the light source and light detector are positioned relative to the inserted cartridge 550 to ensure that the light transmission path between the light source and light detector passes entirely through the fluid patient sample within the cartridge 550 below any free surface of the patient sample that might exist in the cartridge. The light source can emit a consistent and steady light, which can be further standardized by collimating or polarizing the emitted light that is transmitted through the patient sample and received by the light detector. As light is transmitted through the patient sample, components within the patient sample can absorb, scatter, reflect or otherwise affect the incoming light. The light detector therefore registers an altered quantity or characteristic of the light transmitted through the patient sample than light transmitted directly from the light source to the light detector with no intervening patient sample. The altered quantity or characteristic of light transmitted through the patient sample can be included or used during analysis of the patient sample. Optionally, the emitted light from the light source can be divided, such as by a beam splitter. A first portion of the split beam can be passed through the sample to a first light detector and a second portion of the split beam can be directed to a second light detector with no intervening sample. The transmitted light differential can be measured based on the registered transmittance by the first and second light detectors.

The light source can be several different light emitting sources, such as an incandescent bulb, a fluorescent bulb, a light emitting diode (LED), a laser, the sun or other light source. In some example embodiments, the light source can emit a steady light having known characteristics or properties. Alternatively, the light source can emit varied light, such as light emitted by an incandescent bulb. The light source can be modulated to change the intensity and/or wavelength(s) of transmitted light. Such light can be standardized, entirely or in portion, using filters or lenses through which the emitted light is transmitted. For certain analyses, the variance in emitted light properties may not affect the analyses performed, which can be due to the short duration of the analysis or other features of the analysis. An example light source can emit light directly, or with the use of filters and/or lenses, emit light with a wavelength of at least nanometers (i.e., greater than or equal to 650 nm).

The light detector receives light emitted from the light source and then transmitted, refracted or reflected through/from the patient sample. The output from the light detector can be used to quantify or characterize the light received by the detector. Alternatively, the light detector can quantify or characterize the received light itself and output or transmit data or a signal indicative of the quantified/characterized received light. Example light detectors can include photodiodes, digital imaging elements such as a charge coupled device (CCD), a CMOS imager, a photovoltaic array, or other suitable sensors or detectors capable of registering a change in response to received light.

The light source and light detector 506 can be connected to processing circuitry 530 of the reader 500. The processing circuitry 530 can trigger the emission and potentially control the characteristics of light from the light source or receive signals from the light detector based on the quantity and/or characteristics of light received by the light detector.

Reflective surface(s) can be positioned within the housing 502 or positioned relative to the patient sample such that the light emitted from the light source is transmitted multiple times through the patient sample before being received by the light detector. Each of the multiple transmission paths within the patient sample can occur below a free surface of the sample so the entirety of the multiple light transmission path through the sample occurs within the fluid sample. The geometry of the cartridge can assist to ensure that the laser transmission does not extend above any existing free surface of the patient sample.

The repeated transmission of light through the patient sample assists with the analysis of the patient sample. The repeated transmission of the light through the sample increases the transmission path of the light which can correspondingly increase the sensitivity, reliability and/or accuracy of the detected light transmission since the light is transmitted through a larger portion or volume of patient sample and has a higher probability of contacting an element or component within the sample that can result in a change in a property and/or characteristic in the light transmitted through the patient sample.

The reader 500 can include a magnet or magnets 508. The magnet(s) can be included as a portion or part of a MOD, such as the MOD example shown in FIGS. 3A-3B, or used in analysis of a patient sample. The magnet(s) 508 can be movable within the reader 500, allowing the magnet(s) to be moved relative to the patient sample. This can subject the patient sample to the presence of a magnetic field or the presence of a varying magnetic field as the magnet(s) 508 is moved relative to the patient sample. Alternatively, the patient sample can be moved relative to the magnet(s) 508. The magnet 508 can be a permanent magnet and can include a single magnet or multiple magnets. In an example, the magnet includes two permanent magnets, such as the MOD examples taught by U.S. Ser. No. 14/766,523 An example MOD includes two permanent magnets that are positioned on opposite sides of a patient sample, which can also be on opposites sides of a cartridge containing a patient sample. The magnet can also be an electromagnet(s) that can be energized as required or desired during analysis of the patient sample. Further, the strength and polarity of the electromagnet can be varied or set to a required or desired level or orientation.

The reader 500 can include an internal power source 510 that supplies the necessary power to run the components, elements or systems of the reader 500 to perform analysis of patient samples or preserve a minimal, required functionality of the reader. The power source 510 can supply power to the processing circuitry 530, the light source and light detector 506, the magnet 508 or other component, elements or systems of the reader 500. The power source 510 can include one or more batteries or other energy storage devices that provide a required or desired level of power for the reader 500. Additionally, the power source 510 or a portion thereof can be external to the reader 500 and connected thereto as needed or required. External power sources can include batteries or other energy storage devices or a connection to a nearby power source such as a generator, municipal power, or solar array.

The reader 500 can also include pathogen neutralization 512. The pathogen neutralization 512 can include physical components, such as a device or system, or a chemical component. There are many different methods of pathogen neutralization and many different devices/systems capable of performing the methods. The goal of pathogen neutralization is to target specific undesirable biological material, such as diseases and parasites, for destruction/neutralization or to destroy biological material indiscriminately, such as by sterilization. Various systems, such as devices or chemicals that interrupt biological processes or cause the breakdown of biological materials can be to neutralize pathogens within a reader 500 or a cartridge 550.

An ultraviolet (UV) light source is an example pathogen neutralization 512 device that could be used within the reader 500. Exposure to UV light has a debilitating effect on biological material and exposure to intense UV light can cause biological destruction. A UV light source can be placed within the reader 500 and activated to bathe the interior of the reader in UV light, which neutralizes at least a portion of the biological material, including pathogens, within the reader 500. Alternatively, the UV light can be continuously powered on when the reader 500 is in use. The UV light can also be targeted, with one or more UV light sources placed in specific areas of the reader 500 to perform the desired pathogen neutralization. Additionally, the UV light can be positioned to penetrate or bathe a cartridge 550 inserted within the reader 500 to neutralize the patient sample within the cartridge 550 after analysis has been performed. A timing device can be connected to the UV light source to ensure that the UV light source is activated for a necessary amount of time to perform the pathogen neutralization. A photo- or light detector can also be included, such as the light detector of the light source and light detector 506, that can monitor the output of the UV light source to check the continued efficacy of the UV light source or monitor the output of the UV light source to ensure it is activated for a long enough duration to achieve a level of pathogen neutralization. The emitted UV light can affect materials, such as plastic, adversely causing them to become brittle. In some examples, shielding can be included within the housing 502 of the reader 500 to protect areas, components, elements or systems which could be damaged by UV light exposure.

A further pathogen neutralization 512 system can include the use of chemicals to neutralize biological material within the reader 500 or cartridge 550. A chemical based pathogen neutralization 512 system can include the application of chemicals within the reader 500 on a temporary or permanent basis. That is, a chemical application can be applied within the reader 500 during manufacture, the applied chemical application can continuously destroy at least a portion of biological material that contacts a surface upon which the chemical was applied. A temporary chemical based pathogen neutralization 512 system can include a chemical dispersal system that deploys or applies chemicals within the reader 500 or cartridge 550 on actuation, the chemicals contact various surfaces, elements, components or systems of the reader 500, destroying at least a portion of biological material thereon.

In an example embodiment, pathogen neutralizing chemicals, such as a bleach-based solution, can be sprayed, fogged, or distributed about the interior of the reader 500 to perform the pathogen neutralization. The pathogen neutralizing chemicals can be added to the reader 500 by a user, contained within a vessel that is housed, inserted within or fluidically connected to the reader 500. The pathogen neutralizing chemicals, such as the bleach-based solution, can be prepared as needed or can be prepared and stored for later use. An indicator or timer can be included that can indicate to a user once the pathogen neutralization process is complete. The indicator or timer can also prevent the use of the reader 500 until the pathogen neutralization process is complete. As with the previously described pathogen neutralization systems, the chemical-based pathogen neutralization method can also neutralize at least a portion of biological material on or within a cartridge 550 inserted within the reader 500. Additionally, the chemical-based pathogen neutralization chemicals can be pumped or transported through the various components, elements or systems of the reader 500, to disinfect portions that can contact a patient sample, which helps to prevent cross-contamination of patient samples.

An example pathogen neutralization system to neutralize at least a portion of the pathogens of the cartridge 550 can include a portion that is included in the cartridge 550. Pathogen neutralization material, such as powders, fluids or other components can be included in the reader 500 or cartridge 550 assist with neutralization of pathogens within the cartridge 550. The pathogen neutralization material can be included in a portion of the cartridge 550 and dispersed into the collected sample or other portions of the cartridge 550 upon actuation, such as by a user, the reader 500, the cartridge 550, or another source. The pathogen neutralization material can also be integrated with a portion of the cartridge, such as included in the diluent 556. Alternatively, the pathogen neutralization material can be included in the reader 500 and the reader 500 can circulate, or otherwise insert, the pathogen neutralization material into the cartridge 550. The pathogen neutralization material can be targeted to a specific pathogen or be a general wide spectrum pathogen neutralizer.

The reader 500 can include an output 514 that includes one or more visual 516 or audible 518 outputs although in other examples the output is data and does not include visual or audible outputs. The output 514 shown in FIG. 5 communicates information regarding the status of the reader 500, the results of analysis of a patient sample, instructions regarding use of the reader 500 or other information to a user or other computing device. The visual 516 output 514 can include a display, such as a screen, such as a touchscreen, lights, or other visual indicators. The touchscreen used to display information, such as analysis results, to the user can also be used by a user to input to the reader 500. The audible 518 output 514 can include a speaker, buzzer, or other audible indicators. The output 514, visual 516 or audible 518, can be output through an external device, such as a computer, speaker, or mobile device connected physically or wirelessly to the reader 500. The output 514 can output data, including the collected analysis data or interpretative data indicative of the presence or absence of an infection, disease or condition within the patient or the patient sample. An example can include the presence of hemozoin within the patient sample. The interpretive data output can be based on the analysis data collected and processed by the processing circuitry 530 of the reader 500.

The reader 500 can also include temperature control 520. The temperature control 520 can actively or passively control the temperature of at least a portion of the reader 500. Active temperature control 520 can include heating or cooling a portion of the reader 500. Temperature control 520 can also include heating one portion of the reader 500 and cooling another portion of the reader 500. The temperature control 520 can include a refrigeration system, resistive heater, infrared heater, thermoelectric elements, radiator, or other temperature control devices or systems. One example is thermoelectric control of the temperature of the light source which in one example is a laser diode. Passive temperature control can include structures to contain a thermal material in portions of the reader 500. This can include holders for ice, hot water, ice packs, and other thermal materials, the holders retain the thermal material in portions of or about components, elements or systems of the reader 500.

The reader 500 can also include mechanical lysing 522. Mechanical lysing 522 can assist with cell lysing, for example, of cells of a patient blood sample within a cartridge 550 or the lysing of the patient blood sample within the reader 500. Mechanical lysing 522 can include a physical disruptor, or portion thereof, an agitator, a sonicator that can apply sound energy to the patient sample, or other mechanical lysing device or system. The mechanical lysing 522 can interface with or engage the cartridge 550 to facilitate the lysing of the patient sample. The mechanical lysing 522 can be mechanically powered, such as by a wound spring, or electrically powered, such as by a reader 500 power source 510.

The reader 500 can also include a filter 524. The filter 524 can attract, extract, collect or otherwise remove unwanted components or particles in a patient sample of the cartridge 550 or concentrate the wanted components or particles. The filtering of the patient sample by the filter 524 can occur as the patient sample is transferred from the cartridge 550 into the reader 500 or the patient sample can be transferred from the cartridge 550, through the filter 524 and back into the cartridge 550 for analysis. The filter 524 can include structural and chemical features that allow the filter 524 to remove desired or required components from the patient sample. The filter can be affixed in a stationary position to contact the patient sample or moveable through the patient sample to filter the patient sample.

Processing circuitry 530 can be included in the reader 500 to receive input from various components, elements or systems, such as the light source and light detector 506, of the reader 500. The processing circuitry 530 can process the received inputs to perform analysis of the patient sample and output results or data of that analysis. The processing circuitry 530 can include a sample processing module 532, a network module 534, a maintenance module 536 and a database 538. The various elements, 532, 534, 536, 538 and others, of the processing circuitry 530 can be removable or replaceable, allowing replacement and addition of various elements to the processing circuitry 530. In example embodiments, all or a portion of the processing circuitry 530 can be included in the reader 500 and a portion of processing circuitry included in the cartridge 550. The processing circuitry 530 can also control the various components, elements or systems, such as pathogen neutralization 512, mechanical lysing 522, the light source, and others, of the reader 500.

The processing circuitry 530 can initiate or control the analysis of a patient sample within a cartridge 550. The processing circuitry 530 can include preset routines that can be executed by the reader 500 to analyze a patient sample. The preset routines can include prompts for user input or the processing circuitry 530 can prompt a user for input before, during or after analysis of a patient sample. User prompts can include acknowledgement or authorization to proceed through one or more portions of the analysis process. Alternatively, the processing circuitry 530 can initiate, perform, or direct the analysis of the patient sample automatically without user prompts. The processing circuitry 530 can proceed through the various processes and procedures of an analysis of a patient sample, engaging any one or more of the reader 500 systems and collecting the analysis data. The processing circuitry 530 can further automatically process the collected data and transmit a result to a user or other, including an indication the analysis is complete, information regarding the analysis or other indications. The processing circuitry 530 can also transmit the collected data to an external system or device for processing and can transmit a result to the user or the result can be transmitted by one or more of an external system or device.

The sample processing module 532 can receive inputs from the light detector of the light source and light detector 506. Based on the received light detector data, including varying magnetic fields, the sample processing module 532 can determine at least a characteristic of the patient sample, such as a disease or condition, a probability of a characteristic, such as an infection, of the patient sample and quantification of a characteristic, such as a parasite level, of the patient sample. The sample processing module 532 can output an indication of a characteristic, such as an infection, or other various data based on the analysis of the patient sample. The output from the sample processing module 532 can be output through the output 514 of the reader 500 or transmitted to an external device and/or system, such as a computer, mobile device, and remote server or database.

The sample processing module 532 can analyze the patient sample to determine a hemoglobin characteristic, such as a hemoglobin affecting disease or condition, based on the data from various components, elements and/or systems of the reader 500. The results of the analysis can be output from the sample processing module 532 to the output 514 to convey the information to a user or other.

A network module 534 can be included in the processing circuitry 530. The network module can allow the reader 500 to communicate with other readers, computing devices, servers, databases or other devices or systems. The network module 534 can communicate with another device through a physical connection, such as a local area network (LAN), Universal Serial Bus (USB), or wireless, such as Bluetooth^{®}, connection. In an example, the reader 500 can communicate to a remote server through the network module 530 allowing the reader to upload patient sample analysis to the patient's medical records stored on the remote server. The network module 534 can transmit or receive communication to/from the reader 500 and another device or system. In another example, information on the patient can be downloaded to the reader and added to the display or output or used in the analysis(es). For example, demographic information such as age, sex, etc.

A maintenance module 536 can be included in the processing circuitry 530. The maintenance module 536 can perform, initiate or prompt maintenance, calibration, or other processes of the reader 500. Maintenance of the reader 500 can include prompting a user to clean a portion of the reader 500, to replenish resources of the reader 500 and other regular or unscheduled maintenance of the reader 500. Calibration of the reader 500 can include testing components, elements or systems of the reader 500 to check if the reader 500 is in an effective operable state. Additionally, the calibration of the reader 500 can be performed by the maintenance module 536 or prompt a user to perform necessary calibration procedures to allow the reader 500 to perform patient sample analysis effectively and correctly. The maintenance module could also allow automated or semi-automated ordering of supplies or service.

A database 538 can be included in the processing circuitry 530. The database can record patient sample analysis data, patient data, statistical data, test conditions, and other data. The network module 534 can communicate with the database 538 exporting or importing data. The database 538 can be stored on removable or permanent data storage within the reader 500. The database can also occur in whole or in part remote from the reader.

Statistical data of the database 538 can be used during analysis of a patient sample by the sample processing module 532 to assist and/or perform the analysis of a patient sample. This can include tables with reference light transmission amounts or characteristics through various patient samples having determined infections, diseases or conditions and levels of these infections, diseases or conditions. Additionally, the database 538 can include statistical analysis techniques or algorithms that can be used by the sample processing module 532 to determine, calculate or otherwise analyze the patient sample.

The database 538 can also include specific information, such as prior patient analysis results. Such results can be used to determine if the detected condition is new and/or an existing condition. Additionally, the severity of the condition, such as an infection, can be tracked for a particular patient to assess their treatment progress.

The cartridge 550 can contain the patient sample for analysis. The cartridge 550 can be inserted in the cartridge interface 504 and the patient sample analyzed or transferred to the reader 500 for analysis by the components, elements or systems of the reader 500. The cartridge 550 can include a blood collection device or system 552, a filter 554, a diluent 556, a temperature control device or system 558 and a verification element 559.

Blood collection 552 of the cartridge 550 can include a device or system for collecting, storing, or analyzing a patient's blood sample, which can include a passive or active blood collection device or system, a blood sample storage chamber, a blood sample analysis chamber or other chambers, devices or systems to assist or facilitate the collection of a blood sample and analysis of the blood sample.

Active blood sample collection can include the use of a needle, capillary tube or pipette. In an example embodiment, the cartridge 550 can include a needle that can be actuated to deploy from the cartridge 550, piercing a patient's skin and extracting a sample that is drawn into the cartridge 550 and stored for analysis. A further active blood sample collection 552 can be a pipette-like system. The user or other can apply pressure to a bulb or deformable portion of the cartridge 550, the release of pressure on the bulb or deformable portion can draw at least a portion of a patient blood sample into the cartridge 550. The patient can be lanced, poked or pierced to cause bleeding, the blood can be sampled to draw at least a portion of the blood into the cartridge 550 for analysis.

The blood collection 552 can include a lancet or a piercing instrument that can pierce skin to cause bleeding. The blood can be collected using the cartridge 550 to obtain the patient blood sample. Collection of the blood sample can include retraction of the lancet or piercing instrument, carrying a portion go the patient blood into the cartridge 550 for analysis. The blood collection 552 can also include a sealed chamber that is sealed and has negative pressure. A needle can pierce the patient and pierce the sealed chamber, the negative pressure of the sealed chamber causing blood to flow into the sealed chamber due to the pressure differential.

The blood collection 552 can also include a capillary tube or plane that can passively collect a blood sample using capillary action. The patient is caused to bleed, such as by a lancet or other inducing technique, and the capillary tube is placed in the blood to draw a sample into the capillary tube of the cartridge 550 for analysis.

The cartridge 550 can include a filter to filter the patient sample within the cartridge 550. The filter can be placed to filter the patient sample as it is drawn into the cartridge 550 through, before or after, for example, the blood collection 552. In another example, the filter 552 can filter the sample after it has been stored in the cartridge 550. As previously described, the filter can include structural or chemical features to filter a patient sample as necessary or desired.

Diluent 556 to dilute, treat or prepare the patient sample for analysis can be included in the cartridge 550 to be mixed with the collected patient sample. The diluent 556 can be stored in a diluent chamber within the cartridge 550 and separate from the patient sample and mixed automatically or manually. The diluent 556 can be pre-loaded in the same chamber, a mixing chamber or patient sample chamber, that the patient sample will be stored within the cartridge 550. Alternatively, the diluent 556 can be stored in the cartridge 550 remote from the patient sample storage and mixed with the patient sample. The dispensing of the diluent 556 into the patient sample can be triggered manually by the user, or automatically, such as by the cartridge 550 or reader 500. Alternatively, or additionally, the diluent used to prepare the patient sample for analysis can be stored within the reader 500. The reader 500 can add the diluent to the patient sample within the cartridge 550 or can be added to a sample, or mixing, chamber of the reader 500 into which the patient sample, or portion thereof, from the cartridge 550 is transferred. As with the cartridge 550, the sample, or mixing chamber, of the reader 500 can also be pre-loaded with the diluent.

The cartridge 550 can also include temperature control 558, which can include active or passive temperature control systems and/or methods. Passive temperature control 558 can include insulation, structural design features or chemical design features. The passive temperature control 558 can maintain the temperature of the cartridge 550 to preserve a collected patient sample. Active temperature control 558 can include electronic elements, such as thermoelectric elements that can heat or cool at least a portion of the cartridge 550, for example to regulate the temperature of the cartridge 550 or a portion thereof. Temperature control 558 can include heating and/or cooling the temperature of the cartridge before, during or after the collection of a patient sample or the analysis of the sample. The temperature control 558 interfaces with the reader 500 or an external device to regulate the temperature of the cartridge 550.

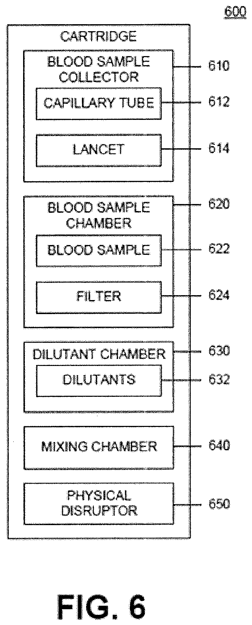

FIG. 6 is a further example cartridge 600, which can include a blood sample collector 610, a blood sample chamber 620, a diluent chamber 630, a mixing chamber 640 and a physical disruptor 650. The various components of the cartridge 600 can be arranged in various configurations depending on the analysis to be performed and other environmental or use considerations. In the example shown in FIG. 6, the cartridges 600 components can be interchangeable allowing a complete cartridge 600 to be assembled from various components.

The blood sample collector 610 of the cartridge 600 can collect a blood sample from a patient. The collector 610 can include devices, components or systems to assist or perform the collection of the blood sample from a patient. The blood sample collector 610 can include a capillary tube/plane 612 or a lancet 614. The capillary tube/plane 612 can use capillary action to draw a blood sample into the cartridge 600. The lancet 614 can be used to pierce, puncture or cut a patient's tissue to cause bleeding, from which a blood sample can be taken.

The collected blood sample 622 can be collected in a blood sample chamber 620 of the cartridge 600. The blood sample chamber 620 can include a filter 624 to filter the blood sample 622. The filter 624 can be positioned within the blood sample chamber 620 of the cartridge 600 such that the blood sample chamber 620 is divided into a first and second portion, which are separated by the filter 624. The blood sample chamber 620 can include structural and/or chemical features to assist with the storage of the blood sample 622 or the analysis of the blood sample 622. Additionally, the blood sample chamber 620 can be located within the cartridge 600 to assist with or facilitate the analysis of the blood sample 622 using a reader.

A diluent chamber 630 storing diluent 632 can be included with the cartridge 600. The diluent 632 within the diluent chamber 630 can be mixed with the blood sample 622 in the blood sample chamber 620 or the cartridge 600 can include a mixing chamber 640 into which the diluent 632 and blood sample 622, or portion(s) thereof, can be mixed before, during or after analysis of the blood sample 622. The diluent 632 can include fluid(s), or substance(s), to dilute the blood sample 622, a reagent, a chemical, a lysing agent, an anti-pathogen agent, an anti-foaming agent, or other fluid(s) that can be assist or facilitate the analysis of the blood sample 622. For example, foaming of the patient sample may compromise the quality of the analyzed data because the bubbles in a foamed patient sample affect the transmission of the light through the sample.

The cartridge 600 can include a physical disruptor 650 that can assist with the lyses of cells of the blood sample 622 in preparation for analysis. The physical disruptor 650 can include a mechanical, optical, or electrical system/device or portion thereof. In an example, a portion of a physical disrupter system or device can be included with the cartridge 600 and the other portion included on the reader and/or another external device. An example physical disruptor 650 can include a sonication horn that can direct sonic energy through the blood sample 622 to assist with lysing of the cells of the blood sample 622. The blood sample can undergo physical disruption in other ways as well, including employing maceration techniques and exposing the blood sample to distilled water or chemicals or any combination of desired disruption techniques.

The lysing can occur before or after dilution or other preparation of the blood sample 622. For example, the cartridge might include elements to transmit the maximum ultrasonic energy to the sample trough rods, cones or other shapes in contact with the blood sample.

The various chambers of the cartridge 600 can be interconnected or in fluid communication, allowing or facilitating the movement or transfer of fluid, with one or more of the chambers of the cartridge 600 or a connection to an external fluid source. The fluid communication between chambers can allow the blood sample 622, the diluent 632 or other fluids to flow or be transferred from chamber to chamber(s) and can include passageways like flexible, rigid, and semi-rigid pipes and tubes. Flow control elements, such as valves, can be positioned along one or more of these passageways to regulate the fluid communication between chambers. The flow control elements can be manually actuated, such as by a reader or user applying pressure to the cartridge 600 or actuating the flow control element, or electrically actuated, such as by a signal from the reader or a user initiated signal or trigger.

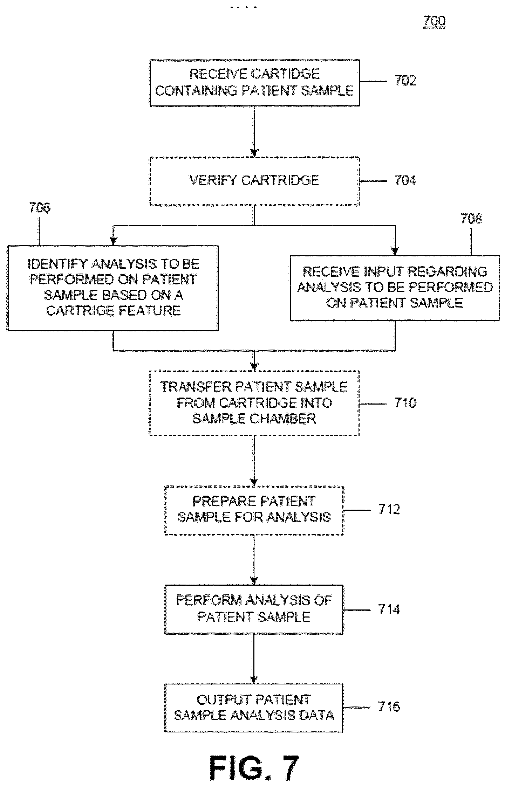

FIG. 7 is an example patient sample analysis process 700 of a reader, processing circuitry, a device or system external to a reader and/or a combination thereof. The reader can receive a cartridge containing a patient sample 702, such as the insertion of a cartridge within a cartridge interface, the reader, or an external device connected to the reader or an external device or system. The cartridge can be optionally verified 704 to determine the validity of the cartridge or the patient sample within. The reader can then identify the analysis to be performed on the patient sample based on a cartridge feature 706, such as structural feature of the cartridge. That is, the reader can recognize or identify the cartridge type and a corresponding analysis that can be performed on the patient sample contained within. Alternatively, the reader can receive an input regarding the analysis to be performed on the patient sample 708. The input can include a user selecting an analysis, communication from an external system or device indicating the analysis to be performed or other input directing the reader to perform an analysis of the patient sample. Optionally, a portion of the patient sample can be transferred from the cartridge into a sample chamber 710 of the reader so that the patient sample can be analyzed within the sample chamber. Additionally, the patient sample can optionally be prepared for analysis 712, which can include lysing the sample, adding a diluent to the patient sample or other preparation performed on or to the patient sample prior to patient sample analysis. The patient sample is then analyzed 714 by the reader and its systems or an external device or system. The patient sample analysis data is then output 716, such as transmitted to a reader or an external device or system. The output 716 can include interpretive data, such as the presence or absence of a disease, infection or condition within the patient sample, including detailed information, such as the type and degree of the disease, infection or condition.

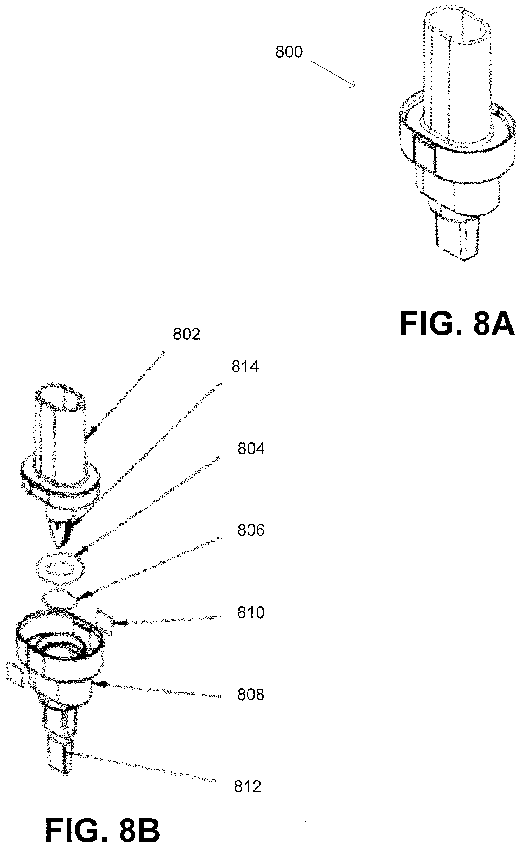

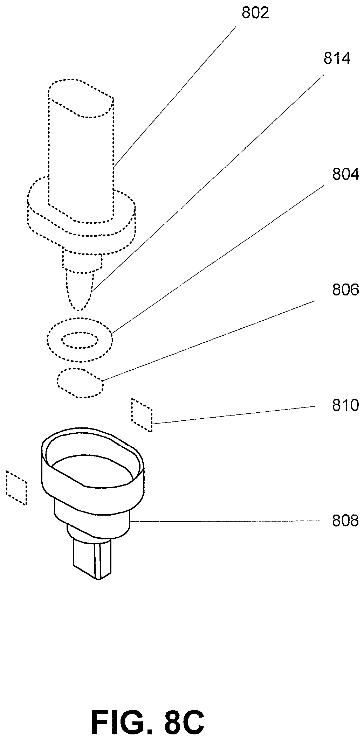

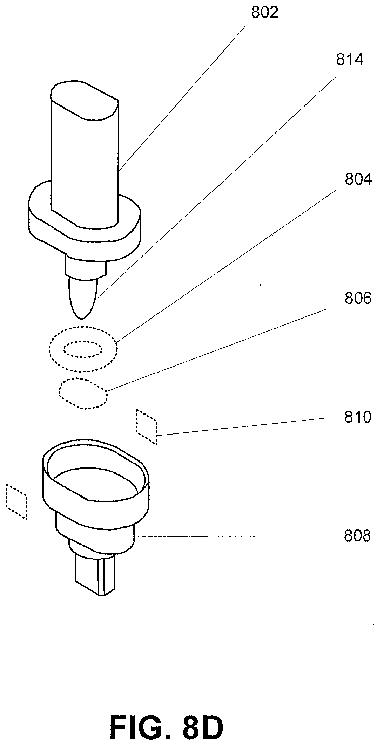

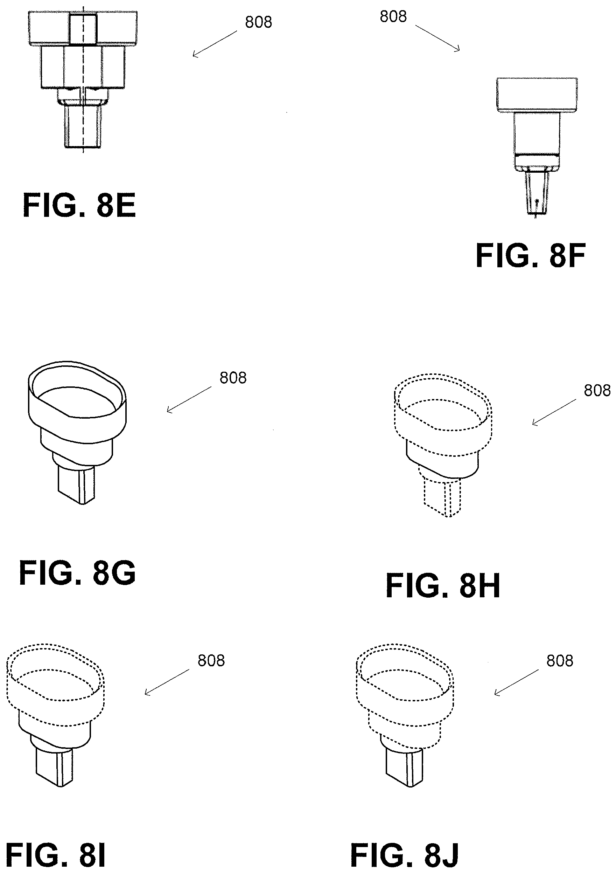

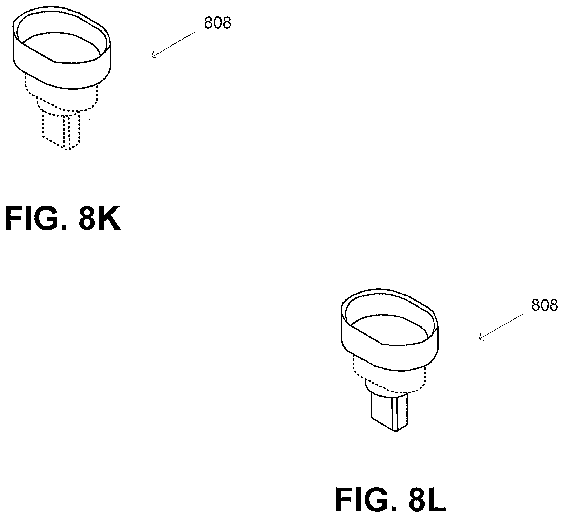

FIGS. 8A-8D illustrate an example cartridge 800. FIG. 8A shows an isometric view of the cartridge 800. FIG. 8B shows an exploded view of the cartridge 800. The cartridge 800 includes a capillary 802 having a capillary plane and first and second ends, an O-ring 804, a membrane 806, and a cuvette 808 having first and second ends. The cartridge 800 can also include at least one of a label 810 or a reagent 812. In one embodiment, the cartridge 800 can be sealed. For example, the cartridge 800 is sealed to the external environment. In yet another example, the cartridge can be sealed permanently or temporarily.

FIGS. 8E-8L illustrate the cuvette 808 of the cartridge 800.

The capillary 802 draws the blood sample into the cartridge 800 via capillary action or captures a fixed volume of a sample from a drop of blood. The capillary plane of the capillary 802 pierces the membrane 806 of the cuvette 808 when the capillary 802 and the cuvette 808 are adjoined, such as during the latching process or the snapping process. The capillary 802 can include a capillary coating 814 to enhance wicking of the sample through the capillary 802. In one embodiment, the sample can be added, such as by pipetting, pouring, or the like, directly into the cuvette 808. The sample can include one or more reagents, one or more microspheres, or one or more reagents and one or more microspheres.

In one embodiment, the capillary 802 and the cuvette 808 are one piece. In one embodiment, the capillary 802 and the cuvette 808 are separate pieces. The cuvette 808, for example, latches with one or more other components of the cartridge 800. Additionally, the capillary 802, for example, latches with one or more other components of the cartridge 800. Alternatively, the cuvette 808 and the capillary 802 can be snapped together, and, once snapped together, do not come apart.

The cuvette 808 is composed of a material, such as glass, crystal, plastic, or combinations thereof, having optical properties allowing for the passage of light of a light source through the cuvette 808 and into the sample to collect information about the sample. For example, the cuvette 808 can be optically clear to allow for the imaging or data collection. In another example, the cuvette 808 can be transparent, semi-transparent, or translucent. In yet another example, the material can be selected based on a certain wavelength to be filtered out or to be passed through to the sample.

The first end of the cuvette 808 can include the membrane 806. The membrane 806 seals the reagent 812 in the cuvette 808 to prevent spillage, for storage purposes, to contain the sample during external sonication, or to be used at a later time. The seal of the membrane 806 can be formed by heat stake with mandrel, hot heat stake with precise volume, hot heat staking with reagent overfill in the cuvette 808, or cold to hot heat staking with reagent overfill in the cuvette 808. Furthermore, the membrane 806 can include a hydrophilic coating, which changes the shape of a meniscus of the reagent 812 during the heat staking process.

The second end of the cuvette 808 can include a fluid chamber which holds the reagent 812, the sample, or a combination thereof. The cuvette 808 can also include a given volume of the reagent 812 or sample. The reagent can include, without limitation, deionized water, surfactants, defoaming agent, stains (e.g., fluorescent, chromogenic, or the like), or the like. In one embodiment, 2% Triton (i.e., a surfactant; a nonionic surfactant that has a hydrophilic polyethylene oxide chain and an aromatic hydrocarbon lipophilic or hydrophobic group) is used as a reagent (e.g., to optimize external sonication), a capillary coating (e.g., to enhance sample collection and wicking), and to release hemozoin, when it is desirous to do so. In another embodiment, a plurality of reagents can be used. For example, a surfactant and a stain can be used.

In one embodiment, the cuvette 808 can also include at least one microsphere to increase mixing, enhance sonication, or increase mixing and enhance sonication. The at least one microsphere can be composed of glass, a polymer, a metal, silica, combinations thereof, or the like. The agitator can range in size from 1 micrometer (µm) to 1 millimeter (mm). For example, 16 0.5 mm glass beads are added to the sample, such as blood, to increase mixing and to enhance sonication. In one embodiment, when two or more microspheres are included, no two microspheres are composed of the same material, such that at least one microsphere is composed of a first material and at least one microsphere is composed of a second material. In another embodiment, when two or more microspheres are included, all of the microspheres are composed of the same material, such that the two or more microspheres are composed of a first material. Alternatively, the at least one microsphere can be added to the sample before being added to the cuvette 808.

The O-ring 804, which can be on the capillary 802 or the cuvette 808, provides a seal between the capillary 802 and the cuvette 808 when the capillary 802 and the cuvette 808 are latched or snapped together. The seal provided by the O-ring 804 can be fluid-tight.

The thickness and shape of the walls of the cartridge 800 are two parameters which impact the transfer of the ultrasonic energy. Therefore, the wall thickness and the wall shape enhance ultrasonic energy transfer or make ultrasonic energy transfer more efficient. Additionally, the sonication probe head 1404 design enhances ultrasonic energy transfer based on the material type of the cartridge 800, the wall thickness of the cartridge 800, the wall shape of the cartridge 800, or combinations thereof.

The label 810 includes a sequence number which can be encoded through an encryption algorithm to a second number. At least one batch number, revision number, or serial number, whether in whole or in part, can be encrypted. The sequence number can also include a manufacturing date to prevent use after an expiration date. The label 810 can also include a design, such as a graphic, code (e.g., QR code), or image, which can be visible to the human eye or only visible to a machine or via another visualization system. The design can include encrypted data.

In one embodiment, one or more machines, such as the external sonication system 1100 or the reader 100, 140, do not work when the number having been encrypted is incorrect (i.e., number being encrypted does not fit a desired or proper format), has been used already, or is not found in a lookup table. This prevents reuse of the cartridge 800 or use of uncertified cartridges. The one or more machines record each cartridge used, such as on internal storage, and upload the sequence numbers to a server or table, which can be internal to the machine or remotely connected, such as in the cloud. The used sequence numbers can be downloaded to all machines when connected to the cloud or remote server to prevent reuse in different machines or to prevent the use of a copied label or sequence number. Alternatively, a remote server can receive all used sequence numbers and identify those sequence numbers that are used more than once, including twice or more. The remote server can compare the sequence number to a database or lookup table. Those used more once, such as twice or more, can be automatically pushed to one or more machines based on location of the machine, frequency of use of the machine, proximity of the attempted counterfeit uses on other machines, or the like. An application, such as one loaded onto the machine or stored in the cloud, can be used to examine data for duplicate sequence numbers and flag them for review, such as by a human or other application.

As another way to prevent reuse, the label 810 or another portion of the cartridge 800 can include at least one of an electrical fused link. As yet another way to prevent reuse, the label 810 or another portion of the cartridge 800 can be marked, scratched, color changed, physically changed (e.g., dimples, pop-ins, or the like), or combinations thereof.

An electronic tag (e.g., IC tag) or RFID tag can be included on the label 810 or another portion of the cartridge 800.

A custom designed cartridge that works with a second device locks into the second device, such that all degrees of freedom are removed to reduce the chance of the fluid sample/medium from vibrating or sloshing, which allows for lower noise and higher sensitivity measurements of the sample volume.

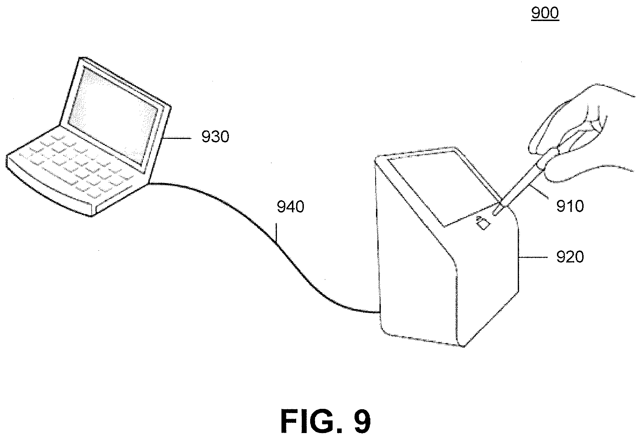

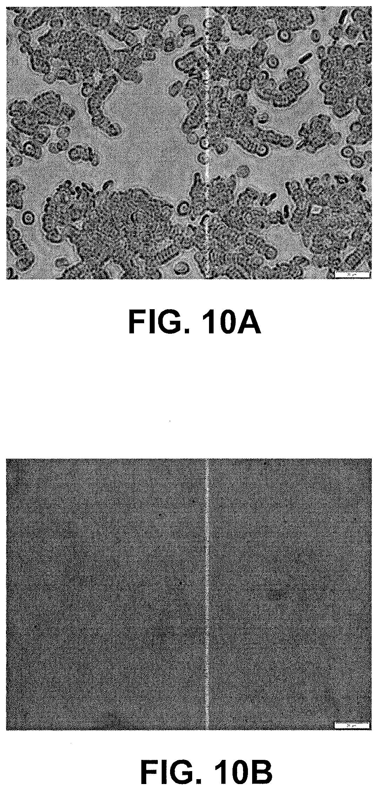

FIG. 9 illustrates an example diagnostic system 900 that includes a cartridge 910 and reader 920, such as described herein, the reader connected 940 to an external device 930, such as a computing device, including a laptop, phone, tablet, a server, remote computer, or other external device. The connection 940 between the reader 920 and the external device 930 can be a physical connection, such as a universal serial bus (USB) connector, such as shown in FIG. 10, or can be a wireless connection, such as an IR, Bluetooth^{®} or WiFi electrical coupling, or a combination thereof. The connection 940 allows communication between the reader 920 and the external device 930. In an example, the reader 920 can perform analysis of a patient sample contained within the cartridge 910, data from the various analysis systems or elements of the reader 920 can be transmitted through the connection 940 to the external device 930 for processing. The external device 930 can then display or transmit the processed results, or portion thereof, to a user or can optionally transmit the processed results back to the reader 920 for display or transmission of the analysis results, or a portion thereof, to the user. In a further example, the reader 920 and external device 930 can both process all or a portion of the patient sample analysis data. The external device 930 can also control one or more aspects of the reader 920, such as the analysis able to be performed by the reader 920, authorized users of the reader 920 or other aspects of the reader 920 and its performance. Additionally, the external device 930 can be in the proximity of the reader 920, such as nearby, or can be remote from the reader 920, such as in another room or in another location including in another country. The external device 930 can communicate with or be connected to multiple readers and or other external systems, such as remote servers or databases.

The reader 920 can be a part of a network. The network can include wired or wireless connections. One or more servers can be incorporated into the network to store or process data and information. Processing can be done remotely. The network can include a plurality of readers, a plurality of servers, a plurality of displays, and the like.

### Sonication/Processing

Sonication is the act of applying sound energy to agitate particles in a sample or medium. During sonication, kinetic energy from the sound oscillation generated from a sonicator creates heat and cavitation bubbles. When this oscillation energy is effectively applied to the sample, the heat and cavitation bubbles (i.e., the forming and bursting of the bubbles) agitate and disrupt the sample components. The volume or size of the sample directly impacts the amount of ultrasonic energy required for total disruption. The fluid viscosity also impacts energy transfer-more viscous samples require more ultrasonic energy.

External sonication is a technique by which ultrasonic energy is applied externally to a cartridge containing the sample. In one embodiment, the cartridge can be sealed. The cartridge used can be one which is commercially available or one which is custom designed, such as the cartridge 800 discussed above.

The ultrasonic energy of external sonication passes through the cartridge walls into the sample, thereby inducing pressure variations and cavitation bubbles result that grow and collapse-the sound waves are transformed into mechanical energy. The mechanical energy that is dissipated into the sample results in effective sample content disruption without requiring any sonication probe in direct contact with the sample fluid. Effective external sonication, for example, maximizes sample content disruption while optimizing energy transfer into the sample, minimizing heat generation in the sample, and minimizing heat and energy dissipation in the external sonication system. External sonication can be used for any appropriate sample. More specifically, some applications of external sonication include, without limitation, working with hazardous sample sources, cellular disruption for virus release in a closed system, DNA fragmentation, and heating/mixing/agitating solutions.

Figure 10A shows a sample before external sonication. Figure 10B shows the sample after external sonication. As can be seen in FIG. 10B, external sonication decreased the opacity of the sample. External sonication disrupts the blood cells of the sample, thereby causing the cellular contents, including the malaria parasite, to be released into the sample medium. The malaria parasite is also disrupted thereby causing the release of the parasite food vacuoles into the sample medium; these food vacuoles are then disrupted which causes the release of hemozoin clumped crystals; the clumped crystals are disrupted by breaking the lipid bonds, thereby resulting in less crystal clumping and individual crystals being freed into the sample medium.

The effectiveness of the external sonication can be further enhanced by optimizing ultrasonic energy transfer efficiency (e.g., frequency, resonate point of an external sonicator system, amount of energy available to be transferred, temperature, amount of time energy is being transferred; by controlling or adjusting the sample volume or the sample viscosity, such as by adding reagents (e.g., deionized water, surfactants, defoaming agent, stains (e.g., fluorescent, chromogenic, or the like), or the like) to the sample to dilute the sample or to reduce residual bubbles within the sample; by adding additional material, as the one or more microspheres, that can seed the start of the cavitation process; by controlling applied voltage of an ultrasonic transducer (fixed or variable); by controlling the current of the ultrasonic transducer (fixed or variable); by controlling the frequency of the ultrasonic transducer (fixed or variable); or by controlling the on/off time of the ultrasonic transducer (fixed or variable). Furthermore, the reagents can be used in downstream processing or are compatible with further downstream processing. In one aspect, a more effective external sonication allows for a reduced ultrasonic energy to be used to disrupt the contents of the sample.

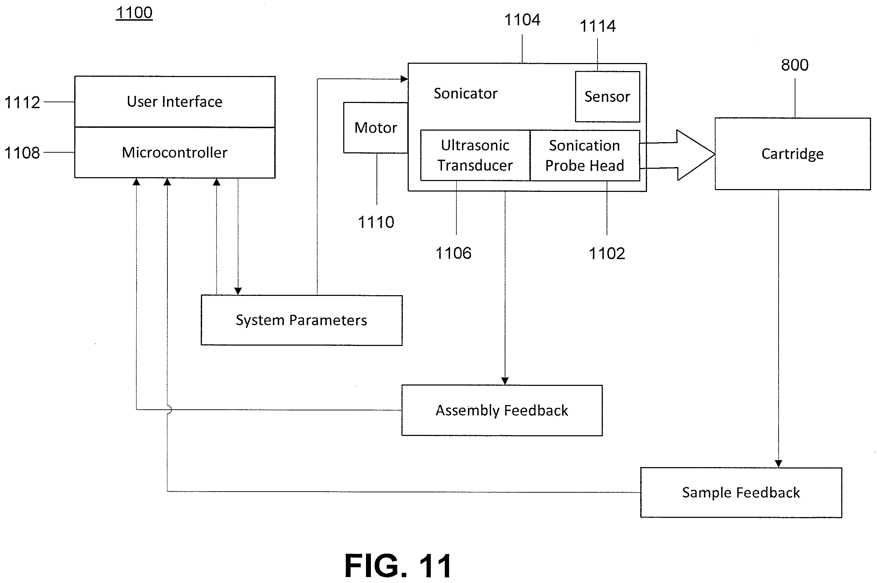

FIG. 11 shows a block diagram for an external sonicator system 1100. First, a sample is provided in the cartridge 800. The sample, prior or subsequent to being added to the cartridge 800, can be processed, such as by adding one or more reagents including, without limitation, magnetic nanoparticles, non-magnetic solids, surfactants, defoaming agent, permeabilizing agents, deionized water, diluents, alcohols (e.g., isopropyl alcohol), biocide, stains/labels, combinations thereof, or the like.

The cartridge 800 is then placed in contact with or is inserted into a probe head 1102 of a sonicator 1104. The sonicator 1104 includes an ultrasonic transducer 1106, which generates ultrasonic energy or ultrasonic sound waves, coupled to the probe head 1102, which transmits the ultrasonic energy or ultrasonic sound waves to the cartridge 800. The ultrasonic energy or ultrasonic sound waves then travel into the sample held within the cartridge 800. To start the transfer of the ultrasonic energy into the cartridge 800, the voltage applied to the ultrasonic transducer 1106 is varied in either a fixed or random frequency around the resonate point of the external sonicator system 1100. In one embodiment, the ultrasonic transducer 1106 is coupled to the probe head 1102 in a manner that does not dampen the available energy for transfer or change the resonate point with, for example, stiffened connections, stiffened supports, dampeners, combinations thereof, or the like.

The probe head 1102 design focuses and increases or decreases energy transfer to and through a wall of the cartridge 800. The design of the probe head 1102 can also be selected to maximize contact area with the cartridge 800. The design of the probe head 1102 transfers ultrasonic energy across one or more surfaces of the cartridge 800 or transfers ultrasonic energy across a plurality of cartridges (i.e., the probe head 1102 can work with a plurality of cartridges, cartridge shapes, cartridge configurations, etc.). For example, the length of the probe head 1102 can be determined by the sonicator frequency or wavelength. As another example, the shape, size, or contact area of the probe head 1102 can be selected to match the one or more characteristics of the walls of the cartridge 800 (e.g., material, thickness, shape, area, radius, curves, corners, combinations thereof, or the like) to optimize or more efficiently focus or disperse the ultrasonic energy. As yet another example, the probe head 1102 matches the resonate point of the ultrasonic transducer 1106. As yet another example, the probe head 1102 can be designed based on two or more of the example discussed above (the length of the probe head 1102 can be determined by the sonicator frequency or wavelength; the shape, size, or contact area of the probe head 1102 can be selected to match the one or more characteristics of the walls of the cartridge 800; and, the probe head 1102 matches the resonate point of the ultrasonic transducer 1106.

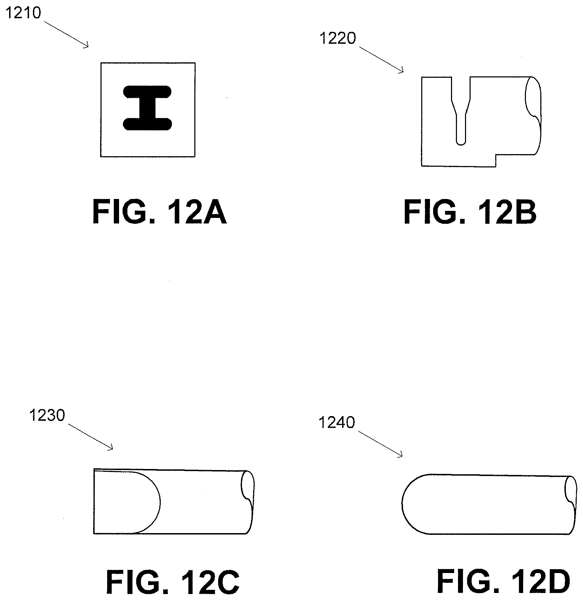

FIGS. 12A-12B show example probe heads into which the cartridge can be inserted. FIGS. 12C-12D show example probe heads that contact the cartridge external surfaces. Example probe heads include a block with an I-shaped cutout extending there through 1210 (FIG. 12A), a block with a tapered, V-shaped, or bottle-shaped cutout extending there through 1220 (FIG. 12B), a partially tapered tip with a flat or chisel-shaped face 1230 (FIG. 12C), or a rod 1240 (12D).

Returning again to FIG. 11, the sonicator 1104 is controlled by a microcontroller 1108 which is programmed to control one or more parameters of the external sonication system 1100 to generate effective content disruption of the sample within the cartridge 800, to determine how the sample changes before, during, and after the sonication, or to provide real time control and feedback. In one embodiment, the microcontroller 1108 is electrically coupled to the ultrasonic transducer 1106 of the sonicator 1104. Variable control, for example, allows for automated adjustments that adjust for potential drift of control parameters during use and over time that would impact disruption effectiveness.

The microcontroller 1108 controls the one or more parameters of the external sonication system 1100 based on at least one of user input, assembly feedback, or sample feedback. The user input can be obtained via a user interface 1112, so as to receive input, such as instructions, sample information, patient information, or the like, or to output results, data, or other information. The user interface can be a display, such as a screen, such as a touchscreen, lights, or other visual indicators. The touchscreen used to display information, such as analysis results, to the user can also be used by a user to input to the external sonication system 1100. An audible output can include a speaker, buzzer, or other audible indicators. The output, visual and/or audible, can be output through an external device, such as a computer, speaker, or mobile device connected physically or wirelessly to the external sonication system 1100. The user interface 1112 can include tactile prompts, input, and output. The output, whether visual, audible, or tactile, can output data, including the collected analysis data and interpretative data indicative of diagnosis, including the presence or absence of an infection, disease, or condition within the patient or the patient sample. An example can include the presence of hemozoin within the patient sample. The interpretive data output can be based on the analysis data collected and processed by processing circuitry of the external sonication system 1100.

The parameters controlled by the microcontroller 1108 and the assembly and sample feedback provided to the microcontroller 1108 include, without limitation, voltage, current, frequency, position, probe head position (e.g., touching or not touching the cartridge), probe head contact force, temperature, time, probe head temperature, sound generation during the external sonication process, sample opacity, sample temperature, and sample agitation. The microcontroller 1108 controls the one or more parameters through hardware or software. For example, the microcontroller 1108 can control temperature of the ultrasonic transducer, the sample, or the ultrasonic transducer and the sample by reducing voltage to the ultrasonic transducer 1106 during the external sonication process; pulsing the voltage on and off to the ultrasonic transducer 1106; or, to running the ultrasonic transducer 1106 until the temperature exceeds a heat threshold, then turning the voltage off to the ultrasonic transducer 1106 until temperature reaches a cool threshold, then repeating until the external sonication process is completed. As another example, the microcontroller 1108 controls the time of external sonication to the ultrasonic transducer, via software or firmware, to continue with the sonication process for an additional amount of time or to discontinue the sonication process at a shorter time duration. Alternatively, though a microcontroller is discussed, the external sonicator system 1100 can include one or more of a processor/microprocessor, memory, or programmable input/output peripherals.

The microcontroller 1108 can also control the movement of the sonicator 1104 along one or more axes by a motor 1110. The motor 1110 can be a servomotor, a stepper motor, an actuator (e.g., piezoelectric, electric, pneumatic, mechanical, linear, or rotary), a solenoid, or any appropriate device for providing motion along at least one axis. In one embodiment, the motor 1110 is coupled to a housing (not shown). In one embodiment, the motor 1110 is coupled to the ultrasonic transducer 1106.

The sonicator 1104 can also include a sensor 1114 to determine the force applied to or on the probe head 1102 or to determine the distance between the probe head 1102 and the cartridge 800 so as to not overtravel (i.e., traveling past a desired point of contact) or under travel (i.e., not traveling to the desired point of contact). The sensor 1114 can be mechanical (e.g., a switch), electrical (e.g., a linear encoder), capacitive, optical (e.g., a laser), acoustic, inductive (e.g., a linear variable differential transformer), or the like.

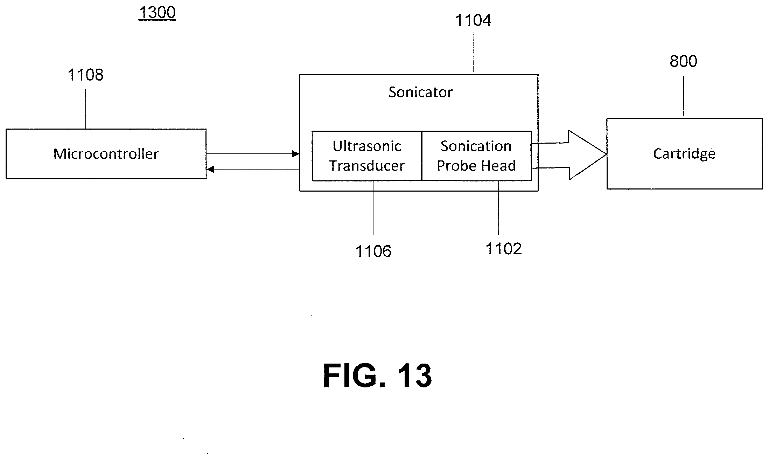

FIG. 13 shows a block diagram for an external sonicator system 1300. The external sonicator system 1300 is similar to the external sonicator system 1100 except the external sonicator system 1300 does not include any system or sample feedback. In one embodiment, the parameters of the external sonication system 1300, as set and controlled by the microcontroller 1108, are fixed (i.e., the parameters do not change). Fixed control does not account for potential drift of control parameters during use and over time that would impact disruption effectiveness. In one embodiment, the parameters of the external sonication system 1300, as set and controlled by a microcontroller 1108, are variable (i.e., one or more of the parameters change). Variable control allows for automated adjustments that adjust for potential drift of control parameters during use and over time that would impact disruption effectiveness.

The external sonicator systems 1100, 1300 can be used in any orientation on the cartridge, such as top sonication, bottom sonication, side sonication, or combinations thereof. Furthermore, the external sonication systems 1100, 1300 can include probe head touch control, such that the external sonication systems automatically turn on when the probe head is in contact with the cartridge and automatically turn off when the probe head is no longer in contact with the cartridge. Alternatively, the external sonication systems can be turned on and off based on a force exerted by or on the probe head.

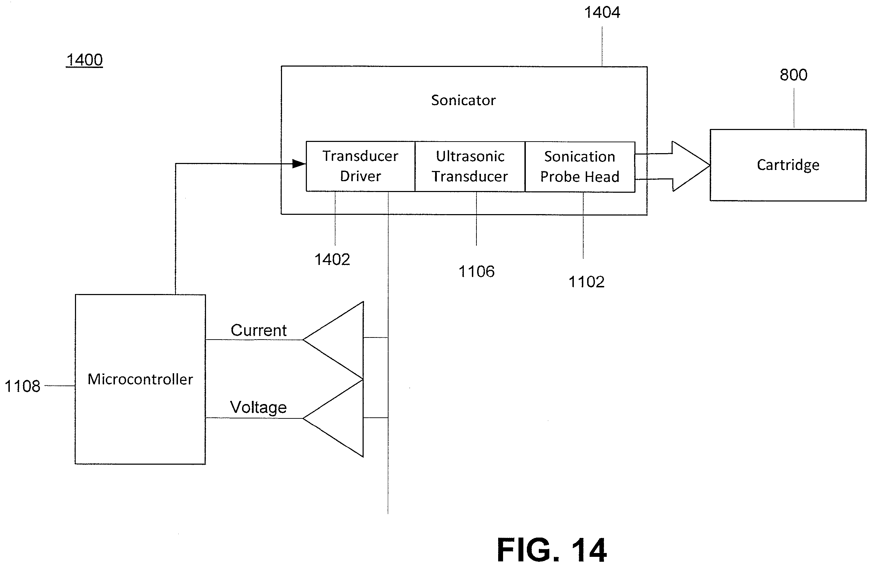

FIG. 14 shows an example driver 1408 including a feedback loop for resonant compensation of an external sonicator system 1400. The driver 1408 defines the maximum power delivery each time and adjusts the frequency based on a closed loop feedback for each sonication cycle as the estimated shift of the resonance point of the ultrasonic transducer 1406 due to effects of pressure/force on the probe head 1402 and also due to contact mechanical resonance of the system considering the ultrasonic transducer 1406 and the cartridge 800.

A method to determine the maximum power delivery each time cycle and adjusting the frequency to compensate for the estimated shift of the resonance point of the ultrasonic transducer 1006 due to effects of pressure force on the probe head 1402 and contact mechanical resonance of the system considering the ultrasonic transducer 1406, the probe head 1402, and the cartridge 800 can also be implemented. The driver 1408, during every pulse start cycle, defines the resonate point of the setup by sweeping through known deviations frequencies and looking for maximum power delivery though a defined proportional-integral-derivative (PID) loop to adjust to the lowest error defined from the theoretical calculation based on LC resonance (i.e., inductor and capacitor) defined by the drive circuitry. The loading effect and a non-functional transducer is detected by measuring the current at a known resonate point which can define a non-functional/over-loaded ultrasonic transducer. The approximation is done by measuring the high side current of the driver circuit and based on statistical data for a given transducer approximates for functional ranges of current draw.

In one embodiment, the sonicator 1404 uses a controlled voltage for the ultrasonic transducer 1406 to control the total energy transfer quotient to the cartridge 800 holding the cells or materials that needs to be lysed or disrupted-as non-controlled drive results in an over-lysing (i.e., burned) sample. The effectiveness of the lysing or disrupting can be at least partially attributed to the overall mixing of the solution and the sample, which is achieved by fast pulsing of the transducer controlled by electronics with effective pulse-width modulation (duty cycle), in order to control total power delivery to the ultrasonic transducer 1406.

For example, a multi-layered piezoelectric element has greater efficacy in breaking the cells at the same frequency and with one-third of the power required to do so.

Performance of the external sonication system 1400 is dependent on operation of the external sonication system 1400 at the resonate point of the external sonication system 1400. Operating the external sonication system 1400 at the resonate point reduces energy loss and heat generation, whereas operating off the resonate point can damage the sample or the external sonication system 1400 due to the generation of excessive heat and sound. The resonate point is impacted by one or more parameters, such as frequency, contact force, surface contact area, and temperature.

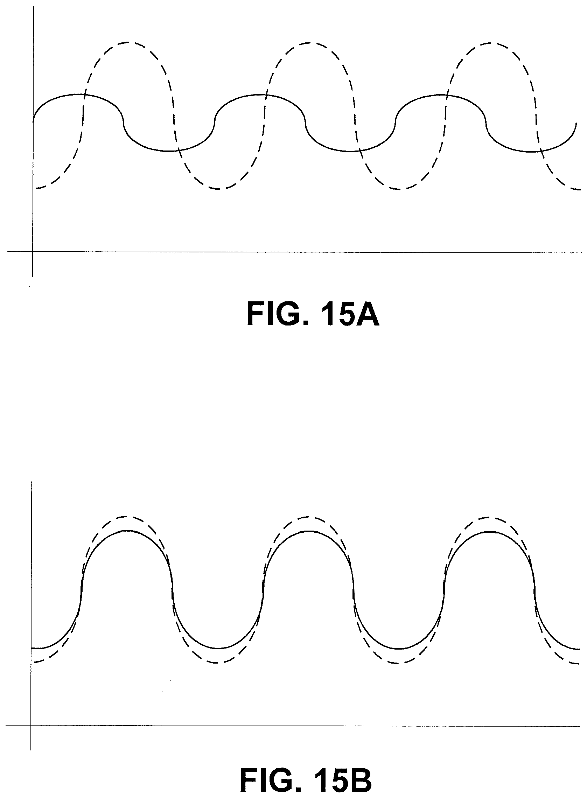

FIGS. 15A-15B show waveforms of the external sonication system 1000. In FIGS. 15A-15B, the solid line represents the measured voltage and the dashed line represents the measured current. In FIG. 15A, the external sonication system 1000 is not operating at the resonate point-the voltage and current are out of phase. Therefore, the energy being delivered is not optimized. In FIG. 15B, the external sonication system 1000 is operating at the resonate point-the voltage and current are in phase. Therefore, the energy being delivered is optimized. The x- and y-axes are dimensionless. In one embodiment, the y-axis can represent amplitude and the x-axis can represent time.

In one embodiment, searching for a resonate point is performed by measuring the current driving the ultrasonic transducer when sweeping across the ultrasonic transducer frequency and voltage. The voltage and frequency which result in the measured peak current, which is the resonate point are used by the drive circuit thereby resulting in the maximum energy deliverable into the cartridge at the lowest energy loss.

Furthermore, the resonate point can be maintained or changed by pre-warming the sonicator 1104 by turning on the ultrasonic transducer 1106 before the sonicator 1104 is brought into contact with the cartridge 800; by pre-loading a contact force of the sonicator 1104 on the cartridge 800 before turning on the ultrasonic transducer 1106; by pulsing the contact force of the sonicator 1104 on the cartridge 800 when turning on the ultrasonic transducer 1106; or, during the external sonication process, using variable contact force of the sonicator 1104 based on measured ultrasonic transducer energy being consumed to optimize energy transfer into the sample.

To measure that the external sonication system is operating within acceptable specifications, changes in the current, temperature, frequency, or combinations thereof can be measured and a threshold can be set to determine when the resonate point of the system has drifted outside of functional specification settings for each of the above.

However, it should be noted that switching between being operating at the resonate point and operating off the resonant can be advantageous. In one aspect, switching between operating on and off the resonate point can increase sample temperature. In another aspect, switching between operating on and off the resonate point can mix or agitate the sample without heating or causing disruption of the contents of the sample.

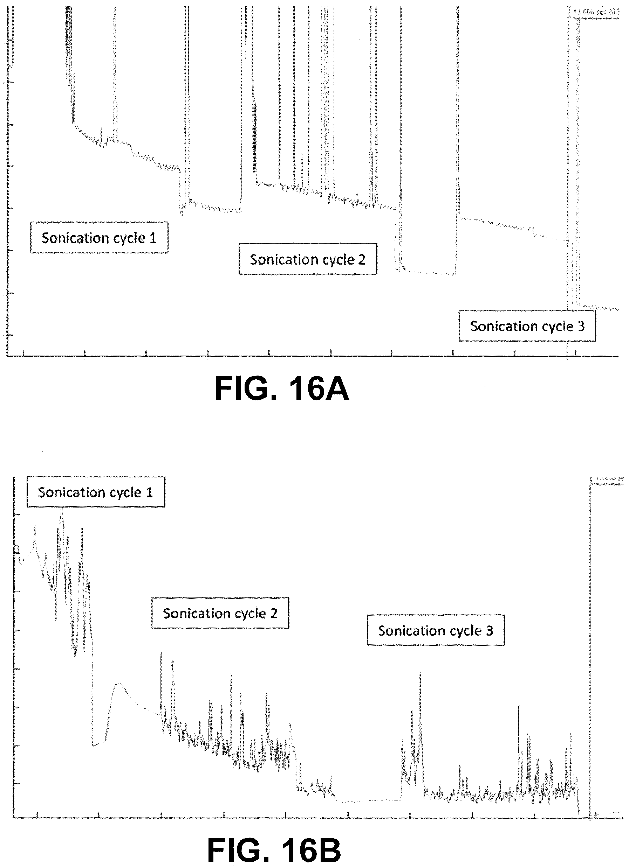

FIGS. 16A-16B illustrate waveforms showing non-desired sonication and desired sonication, respectively. To determine whether the sonication is desirable or non-desirable, sample opacity can be measure before, during, and after sonication. Alternatively, or in addition to the measurements, one or more algorithms can be employed which determined absolute or relative changes in the sample opacity before sonication and after sonication, and sample opacity change during sonication.

As shown in FIG. 16A, when measuring changes in sample opacity before, during, and after the external sonication process a very small change in voltage is observed (i.e., < 30mV) and very little change of signal from the sample is observed during the external sonication process. This waveform signature typifies undesirable external sonication of the sample as there is minimal change in opacity and minimal opacity agitation during the external sonication process. This information is then used by the system to determine if additional external sonication should be applied or testing should proceed. In the example provided in FIG. 16A, additional external sonication would be performed or a notification would be provided that the external sonication failed, such that the sample would not proceed to the next step-at least until proper external sonication has occurred. When the external sonication, as performed, provides non-desirable results, the external sonication can be repeated and the control parameters can be adjusted to achieve desirable results.

As shown in FIG. 16B, the waveform signature typifies desirable external sonication in the sample as there is a large change in opacity and large opacity agitation during sonication process. In this example, the system measures changes taking place within the sample before, during, and after the external sonication process. In this example, when measuring changes in sample opacity before, during, and after external sonication, a very large change in voltage is observed (i.e., > 300mV, which is 10x the change observed in FIG. 16A) and very large change of signal from sample is observed during the external sonication process. This information is then used by the system to determine if additional external sonication should be applied or testing should proceed. In the example provided in FIG. 16B, additional external sonication need not be performed and the sample can proceed to the next step of testing or processing.

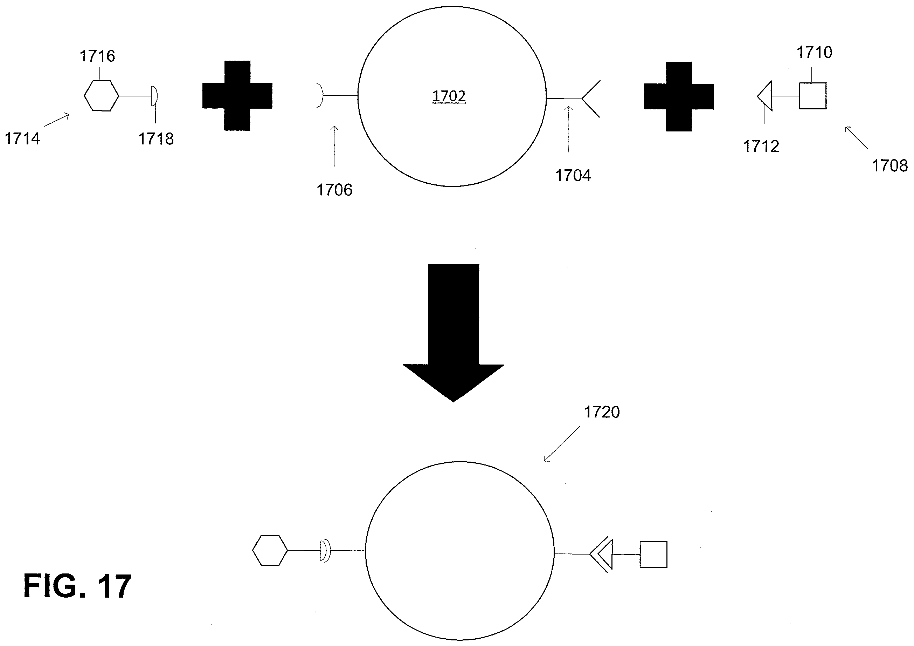

Target materials that are unresponsive to the applied magnetic field in their normal state can be processed so that the materials respond to the applied magnetic field. This allows for the collection of as much diagnostically-relevant information as possible. FIG. 17 illustrates an example target material 1702 having been conjugated to a magnetic nanoparticle 1710 and a non-magnetic solid 1716, thereby forming a complex 1720 that is magnetic and capable of light-blocking. The magnetic nanoparticle 1710 and the non-magnetic solid 1716 react differently to the applied magnetic field to allow for detection of one or more complexes at the same time within the same sample. The non-magnetic solid 1716 can be three-dimensional. For example, the shape, size, and composition of the non-magnetic solid 1716 affect the speed of alignment, the speed of the migration of the complex 1720, to affect the absorption/transmission of light at different wavelengths, or the like. The magnetic nanoparticle 1710 can be paramagnetic or ferromagnetic. The magnetic nanoparticle 1710 allows for the non-magnetic target material 1702 to respond to the applied magnetic field.

The signal characteristics detected within the system can be used to determine the target material 1702 present within the sample. For example, a first target material conjugated to a spherical non-magnetic solid migrates at a first rate, whereas a second target material conjugated to dodecahedron migrates at a second rate. If the first rate of migration is calculated within the sample, then the first target material is present within the sample. If the second rate of migration is calculated within the sample, then the second target material is present within the sample. Furthermore, if two migration rates are calculated within the sample, then the first and second target materials are both present within the sample.

To form the complex 1720, a first affinity molecule-magnetic nanoparticle conjugate 1708 and a second affinity molecule-non-magnetic solid conjugate 1714 are added to the sample. The first affinity molecule-magnetic nanoparticle conjugate 1708 includes the magnetic nanoparticle 1710 conjugated to a first affinity molecule 1712, which recognizes and binds to a first biomarker 1704 of the target material 1702. The second affinity molecule-non-magnetic solid conjugate 1714 includes the non-magnetic solid 1716 and a second affinity molecule 1718, which recognizes and binds to a second biomarker 1706 of the target material 1702.

Though the first and second affinity molecules 1712, 1718 are discussed as recognizing and binding to first and second biomarkers 1704, 1706, respectively, the first and second affinity molecules 1712, 1718 can, in one embodiment, recognize and bind to the same biomarker. In another embodiment, more than two affinity molecules can be used to recognize and bind to more than two biomarkers, such as when it is desirous to detect more than one target material or to detect a target material having one or more biomarkers different than other target material.

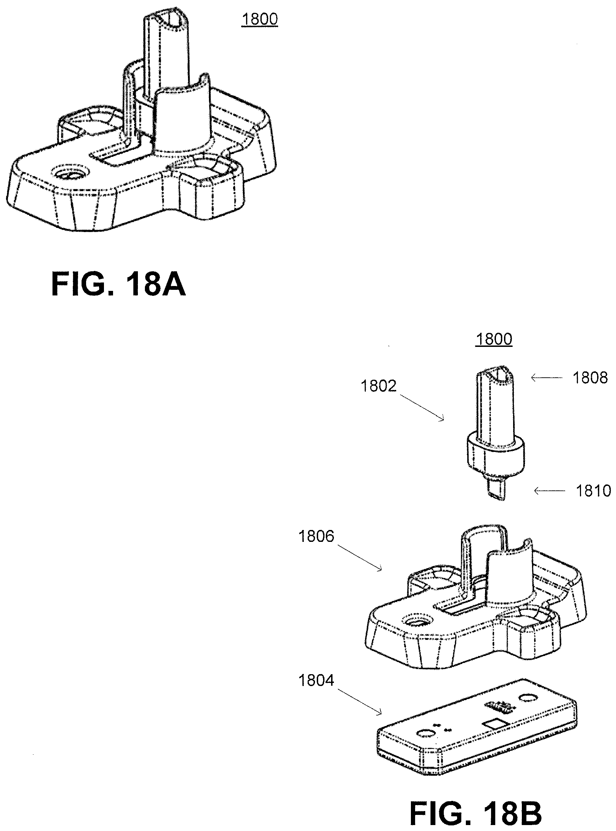

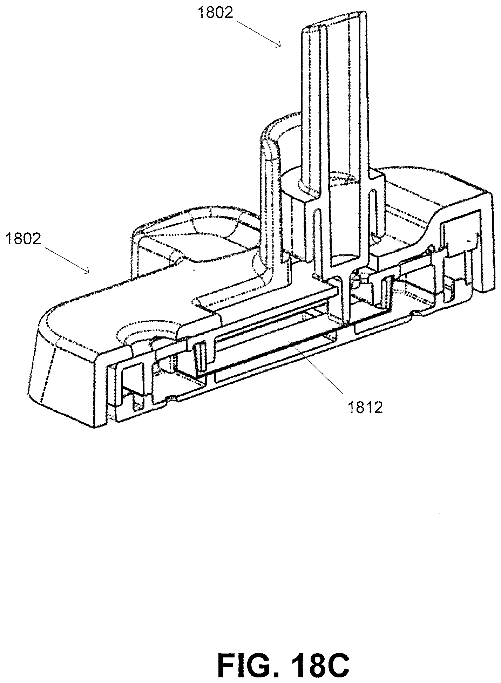

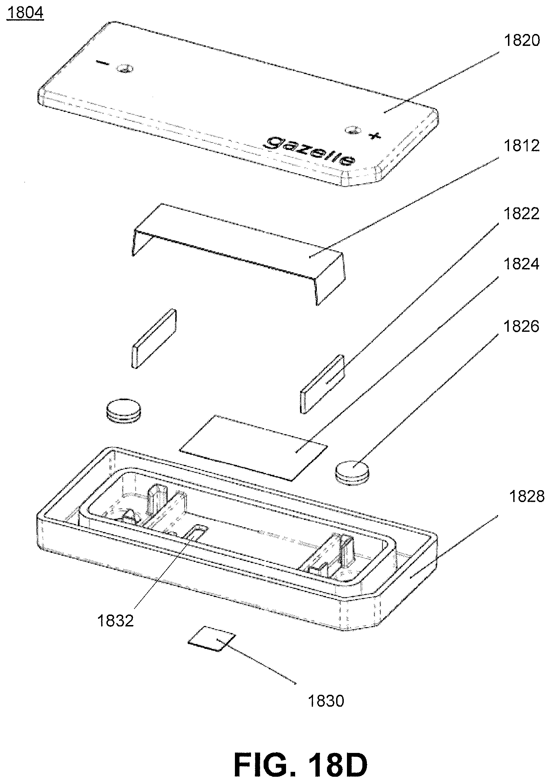

A system and method for transferring a sample or portion thereof from a cartridge to a substrate, such as for further processing, handling, or storage, is contemplated. FIGS. 18A-18C illustrate a printer 1800 that aligns a stamper 1802 with a cartridge 1804, as shown in FIG. 18D, via a stand 1806 to transfer or print a sample or portion thereof onto a substrate 1812 housed within the cartridge 1804. The stamper 108 includes a tip 1810 at a first end and a second end 1808. In one embodiment, the tip 1810 includes an opening and is hollow and fluidly connected to a cavity in the second end 1808, thereby allowing for a sample to be added to the cavity and dispensed, transferred, or printed via an opening in the tip 1810. In one embodiment, the tip 1810 is solid or does not include an opening, such that the sample dispensed, transferred, or printed by touching the tip 1810 to the sample and then touching the tip to the substrate 1812.

Examples of the substrate 1812 can be a slide, a well plate, an electrophoresis gel, or cellulose acetate paper. In one embodiment, the stamper 1802 collects the sample, such as a drop of blood from a finger prick, such as having used a lancet. In another embodiment, the stamper 1802 collector the sample from a slide or well plate, such as used in laboratory setting.

In one embodiment, when the substrate 1812 is the electrophoresis gel or cellulose acetate paper, the sample transferred to the substrate 1812 can undergo subsequent electrophoresis (i.e., applying fixed or variable current or voltage causing the sample components to migrate and separate across or along the substrate 1812) to, for example, separate and detect hemoglobin variants. In one embodiment, cellulose acetate paper can be wetted before the sample is printed or transferred. In one embodiment, the sample includes a mixture of the patient blood that has been lysed and mixed with a marker fluid.

Internal features of the stamper 1802, such as protrusions, ribs, flanges, guides, slits, or the like, align to the top corners of the cartridge 1804. External ribs on the stamper 1802 align to the sides of the stand 1806. These features control side-to-side and fore-to-aft positions.

When the cartridge 1804, the stamper 1802, and the stand 1806 are assembled without any force applied, the stamper 1802 is aligned with the cartridge 1806 but held slightly above the substrate 1812 by a snap dome. The snap dome provides positive feedback that the sample or portion thereof has been transferred to or printed on the substrate 1812. The stamper 1802 contacts the substrate 1812 when sufficient vertical force is applied to overcome the resistance provided by the snap dome. The force can be applied directly to the stamper 1802 or to the stand 1806. In either case, the sample or portion thereof is transferred from the stamper 1802 to the substrate 1812.

The graphic pattern of the sample or portion thereof is defined by the shape of the tip 1810. The shape of the tip 1810 can be a square, rectangle, circle, line, triangle, or any appropriate shape. Furthermore, the stamper can act as stand for the cartridge. In one embodiment, the cartridge is located upright in a hole in the top of the stamper having a shape corresponding to the shape of the cartridge.

The cartridge 1804 includes a first housing piece 1820, the substrate 1812, and a second housing piece 1828. The first housing piece 1820 can include one or more holes through which leads for a power supply can be inserted to engage one or more electrodes 1826, such as for electrophoresis. The second housing piece 1828 includes an aperture 1832 through which the tip 1810 is inserted to dispense, transfer, or print the sample. The first housing piece 1820 and the second housing piece 1828 can be welded together, such as by ultrasonic welding, snapped together, latched together, adjoined by a joint (e.g., dovetail, tongue and groove), adjoined by a fit press, adjoined by an adhesive, or the like.

The one or more electrodes 1826 can be press fit or adhered to the first or second housing pieces 1820, 1828. The cartridge 1804 can also include at least one or a blotter paper 1822, an anti-fog window 1824, or a label 1830.

The printer 1800 and the components thereof can be composed of metal, plastic, glass, ceramic, combinations thereof, or the like.

Though certain elements, aspects, components or the like are described in relation to one embodiment or example, such as an example external sonication system, those elements, aspects, components or the like can be including with any other external sonication system, such as when it desirous or advantageous to do so.

The foregoing description, for purposes of explanation, used specific nomenclature to provide a thorough understanding of the disclosure. However, it will be apparent to one skilled in the art that the specific details are not required in order to practice the systems and methods described herein. The foregoing descriptions of specific embodiments are presented by way of examples for purposes of illustration and description. They are not intended to be exhaustive of or to limit this disclosure to the precise forms described. Many modifications and variations are possible in view of the above teachings. The embodiments are shown and described in order to best explain the principles of this disclosure and practical applications, to thereby enable others skilled in the art to best utilize this disclosure and various embodiments with various modifications as are suited to the particular use contemplated. It is intended that the scope of this disclosure be defined by the following claims.

## Claims

1. An external sonication system (100) for disrupting a sample or component thereof, the system having a resonate point frequency, and comprising:
a cartridge (120) to hold the sample, the cartridge comprising
a sample chamber (240), and
at least one wall forming the sample chamber;
a sonicator (1104) comprising an ultrasonic transducer (1106) coupled to a probe head (1102); and,
a microcontroller (1108) electrically coupled to the sonicator, wherein the microcontroller is programmed to control the frequency of the ultrasonic transducer based on feedback to ensure that the system is operating at the resonate point frequency of the external sonification system,
wherein the probe head contacts an external surface of the at least one wall of the cartridge with a probe head contact force and does not contact the sample,
wherein the feedback includes the probe head contact force.

2. The system (100) of claim 1, wherein the ultrasonic transducer (1106) is coupled to the probe head (1102) with a non-dampening or non-resonant-point-changing pressure or force.

3. The system (100) of claim 1, wherein the microcontroller (1108) is programmed to determine resonate point drift outside of a functional frequency by calculating changes to current, voltage, frequency, or one or more combinations thereof.

4. The system (100) of claim 1, wherein the microcontroller (1108) is programmed to control a position and a touch force of the sonicator (1104).

5. The system (100) of claim 1, wherein the microcontroller (1108) is programmed to cause the start of the transfer of the ultrasonic energy into the cartridge (120) by varying the voltage applied to the ultrasonic transducer (1106) in a fixed or random frequency proximal to the resonate point frequency.

6. The system (100) of claim 1, wherein the microcontroller (1108) is programmed to control the ultrasonic transducer (1106) by at least one of:
reducing voltage supplied to the ultrasonic transducer,
pulsing the voltage supplied to the ultrasonic transducer on and off, or
causing the ultrasonic transducer to receive the supplied voltage until an ambient or ultrasonic transducer temperature exceeds an active threshold, then upon reaching the temperature threshold, causing the ultrasonic transducer to terminate receiving the supplied voltage until the ambient or ultrasonic temperature reaches a resting threshold.

7. The system (100) of claim 1, wherein the microcontroller (1108) is programmed to search for the resonate point by measuring the current driving the ultrasonic transducer (1106) when sweeping across the ultrasonic transducer frequency and voltage.

8. The system (100) of claim 1, wherein the microcontroller (1108) is programmed to maintain or to change the resonate point by at least one of:
pre-warming the sonicator (1104) by turning on the ultrasonic transducer (1106) before the sonicator is brought into contact with the cartridge (120),
pre-loading a contact force of the sonicator on the cartridge before turning on the ultrasonic transducer,
pulsing the contact force of the sonicator on the cartridge when turning on the ultrasonic transducer, or
using variable contact force of the sonicator based on measured ultrasonic transducer energy being consumed to optimize energy transfer into the sample.

9. The system (100) of claim 1, wherein the microcontroller (1108) is programmed to alternate between mixing or agitating the sample at a frequency on the resonate point frequency and off the resonate point frequency.

10. The system (100) of claim 1, wherein the microcontroller (1108) is programmed to determine sonication effectiveness by performing at least one of:
measuring sample opacity before sonication and comparing the measured sample opacity against an opacity value,
measuring the sample opacity during sonication and comparing the measured sample opacity against the opacity value,
measuring the sample opacity after sonication and comparing the measured sample eopacity against the opacity value, or
determining absolute or relative changes in the sample opacity based on absolute or relative values, respectively, in the sample opacity taken before and after sonication.

11. The system (100) of claim 10, wherein the microcontroller (1108) is programmed to receive a measurement of sample opacity, and output a failed sonication alert when the measurement of sample opacity does not meet or exceed a sample opacity threshold value.

12. The system (100) of claim 1, wherein the microcontroller (1108) is programmed to determine resonate point drift outside of a functional specification by
measuring changes to current, temperature, and frequency; and
comparing the current, temperature and frequency change measurements to a threshold.

13. The system (100) of claim 1, wherein the microcontroller (1108) is programmed to determine a maximum power delivery each time cycle and to adjust a frequency to compensate for an estimated shift of the resonate point frequency of the ultrasonic transducer (1106) due to effects of pressure force on the probe head (1102) and contact mechanical resonance.

14. The system (100) of claim 13, wherein the microcontroller (1108) is further programmed to sweep through, at the start of every pulse cycle, known deviation frequencies, and determine maximum power delivery though a defined proportional-integral-derivative (PID) loop to adjust to the lowest error based on inductor-capacitor resonance of a drive circuitry.

15. The system (100) of claim 14, wherein the microcontroller is further programmed to identify a non-functioning or overloaded ultrasonic transducer (1106) by measuring a high side current of a drive circuitry and comparing the high side current to current data of the ultrasonic transducer.

## Patentansprüche

1. Externes Beschallungssystem (100) zum Aufbrechen einer Probe oder einer Komponente davon, wobei das System eine Resonanzpunktfrequenz aufweist, und Folgendes umfasst:
eine Kartusche (120) zur Aufnahme der Probe, wobei die Kartusche umfasst
eine Probenkammer (240), und
mindestens eine die Probenkammer bildende Wand;
einen Sonikator (1104), der einen an einen Sondenkopf (1102) gekoppelten Ultraschallwandler (1106) umfasst; und,
einen Mikrocontroller (1108), der elektrisch mit dem Sonikator gekoppelt ist, wobei der Mikrocontroller programmiert ist, die Frequenz des Ultraschallwandlers basierend auf Rückkopplung zu regeln, um sicherzustellen, dass das System bei der Resonanzpunktfrequenz des externen Beschallungssystems arbeitet,
wobei der Sondenkopf mit einer Kontaktkraft des Sondenkopfes eine Außenfläche der mindestens einen Wand der Kartusche kontaktiert und nicht mit der Probe in Kontakt tritt,
wobei die Rückkopplung die Kontaktkraft des Sondenkopfes einschließt.

2. System (100) nach Anspruch 1, wobei der Ultraschallwandler (1106) mit einem Druck oder einer Kraft an den Sondenkopf (1102) gekoppelt ist, die nicht dämpfend ist oder den Resonanzpunkt nicht verändert.

3. System (100) nach Anspruch 1, wobei der Mikrocontroller (1108) programmiert ist, um eine Drift des Resonanzpunkts außerhalb einer Funktionsfrequenz zu bestimmen, indem er Änderungen von Strom, Spannung, Frequenz oder einer oder mehreren Kombinationen davon berechnet.

4. System (100) nach Anspruch 1, wobei der Mikrocontroller (1108) programmiert ist, um eine Position und eine Berührungskraft des Sonikators (1104) zu regeln.

5. System (100) nach Anspruch 1, wobei der Mikrocontroller (1108) programmiert ist, um den Beginn der Übertragung der Ultraschallenergie in die Kartusche (120) dadurch zu bewirken, dass die an den Ultraschallwandler (1106) angelegte Spannung mit einer festen oder zufälligen Frequenz in der Nähe der Resonanzpunktfrequenz variiert wird.

6. System (100) nach Anspruch 1, wobei der Mikrocontroller (1108) programmiert ist, um den Ultraschallwandler (1106) durch mindestens eines von Folgendem zu regeln:
Verringern der dem Ultraschallwandler zugeführten Spannung,
Takten der dem Ultraschallwandler zugeführten Spannung ein und aus, oder
Veranlassen, dass der Ultraschallwandler die zugeführte Spannung erhält, bis eine Umgebungs- oder Ultraschallwandler-Temperatur einen aktiven Schwellenwert überschreitet, und dann beim Erreichen des Temperaturschwellenwerts Veranlassen, dass der Ultraschallwandler den Empfang der zugeführten Spannung beendet, bis die Umgebungs- oder Ultraschalltemperatur einen Ruhe-Schwellenwert erreicht.

7. System (100) nach Anspruch 1, wobei der Mikrocontroller (1108) programmiert ist, nach dem Resonanzpunkt zu suchen, indem der Strom gemessen wird, der den Ultraschallwandler (1106) antreibt, wenn die Frequenz und die Spannung des Ultraschallwandlers durchfahren werden.

8. System (100) nach Anspruch 1, wobei der Mikrocontroller (1108) programmiert ist, um den Resonanzpunkt durch mindestens eines von Folgendem aufrechtzuerhalten oder zu verändern:
Vorwärmen des Sonikators (1104), indem der Ultraschallwandler (1106) eingeschaltet wird, bevor der Sonikator mit der Kartusche (120) in Kontakt gebracht wird,
Vorbelasten einer Kontaktkraft des Sonikators auf die Kartusche, bevor der Ultraschallwandler eingeschaltet wird,
Takten der Kontaktkraft des Sonikators auf die Kartusche beim Einschalten des Ultraschallwandlers, oder
Verwenden einer variablen Kontaktkraft des Sonikators basierend auf einer gemessenen vom Ultraschallwandler verbrauchten Energie, um die Energieübertragung in die Probe zu optimieren.

9. System (100) nach Anspruch 1, wobei der Mikrocontroller (1108) programmiert ist, um abwechselnd die Probe bei einer Frequenz auf der Resonanzpunktfrequenz und bei einer Frequenz außerhalb der Resonanzpunktfrequenz zu mischen oder aufzurühren.

10. System (100) nach Anspruch 1, wobei der Mikrocontroller (1108) programmiert ist, um die Wirksamkeit der Beschallung durch mindestens eines von Folgendem zu bestimmen:
Messen der Probenopazität vor der Beschallung und Vergleichen der gemessenen Probenopazität mit einem Opazitätswert,
Messen der Probenopazität während der Beschallung und Vergleichen der gemessenen Probenopazität mit dem Opazitätswert,
Messen der Probenopazität nach der Beschallung und Vergleichen der gemessenen Probenopazität mit dem Opazitätswert, oder
Bestimmen absoluter oder relativer Änderungen der Probenopazität basierend auf absoluten bzw. relativen Werten in der vor und nach der Beschallung erfassten Probenopazität.

11. System (100) nach Anspruch 10, wobei der Mikrocontroller (1108) programmiert ist, um eine Messung der Probenopazität zu empfangen und eine Warnmeldung für fehlgeschlagene Beschallung auszugeben, wenn die Messung der Probenopazität einen Schwellenwert der Probenopazität weder erreicht noch überschreitet.

12. System (100) nach Anspruch 1, wobei der Mikrocontroller (1108) programmiert ist, um eine Drift des Resonanzpunkts außerhalb einer Funktionsspezifikation durch Folgendes zu bestimmen
Messen von Änderungen des Stroms, der Temperatur und der Frequenz; und
Vergleichen der Strom-, Temperatur- und Frequenzänderungsmessungen mit einem Schwellenwert.

13. System (100) nach Anspruch 1, wobei der Mikrocontroller (1108) programmiert ist, um in jedem Zeitzyklus eine maximale Leistungsabgabe zu bestimmen und eine Frequenz anzupassen, um eine geschätzte Verschiebung der Resonanzpunktfrequenz des Ultraschallwandlers (1106) aufgrund von Wirkungen einer Druckkraft auf den Sondenkopf (1102) und einer Kontaktmechanik-Resonanz zu kompensieren.

14. System (100) nach Anspruch 13, wobei der Mikrocontroller (1108) weiter so programmiert ist, dass er zu Beginn jedes Pulszyklus bekannte Abweichungsfrequenzen durchläuft und eine maximale Leistungsabgabe durch eine definierte Proportional-Integral-Differential-(PID)-Schleife bestimmt, um basierend auf einer Induktor-Kondensator-Resonanz einer Antriebsschaltung eine Anpassung an den kleinsten Fehler vorzunehmen.

15. System (100) nach Anspruch 14, wobei der Mikrocontroller weiter so programmiert ist, dass er einen nicht funktionierenden oder überlasteten Ultraschallwandler (1106) identifiziert, indem ein High-Side-Strom einer Antriebsschaltung gemessen und der High-Side-Strom mit Stromdaten des Ultraschallwandlers verglichen wird.

## Revendications

1. Système de sonication externe (100) pour désagréger un échantillon ou un composant de celui-ci, le système présentant une fréquence du point de résonance, et comprenant :
une cartouche (120) destinée à contenir l'échantillon, la cartouche comprenant
une chambre d'échantillon (240), et
au moins une paroi formant la chambre d'échantillon ;
un sonicateur (1104) comprenant un transducteur ultrasonore (1106) couplé à une tête de sonde (1102) ; et,
un microcontrôleur (1108) électriquement couplé au sonicateur, dans lequel le microcontrôleur est programmé pour commander la fréquence du transducteur ultrasonore sur la base d'un retour d'information afin de garantir que le système fonctionne à la fréquence du point de résonance du système de sonication externe,
dans lequel la tête de sonde vient en contact avec une surface externe de ladite au moins une paroi de la cartouche avec une force de contact de tête de sonde et ne vient pas en contact avec l'échantillon,
dans lequel le retour d'information inclut la force de contact de tête de sonde.

2. Système (100) selon la revendication 1, dans lequel le transducteur ultrasonore (1106) est couplé à la tête de sonde (1102) avec une pression ou une force non amortissante ou ne modifiant pas le point de résonance.

3. Système (100) selon la revendication 1, dans lequel le microcontrôleur (1108) est programmé pour déterminer une dérive du point de résonance en dehors d'une fréquence de fonctionnement en calculant des variations de courant, de tension, de fréquence, ou une ou plusieurs combinaisons de ceux-ci.

4. Système (100) selon la revendication 1, dans lequel le microcontrôleur (1108) est programmé pour commander une position et une force de contact du sonicateur (1104).

5. Système (100) selon la revendication 1, dans lequel le microcontrôleur (1108) est programmé pour provoquer le début du transfert de l'énergie ultrasonore dans la cartouche (120) en faisant varier la tension appliquée au transducteur ultrasonore (1106) à une fréquence fixe ou aléatoire à proximité de la fréquence du point de résonance.

6. Système (100) selon la revendication 1, dans lequel le microcontrôleur (1108) est programmé pour commander le transducteur ultrasonore (1106) par au moins l'une des opérations suivantes :
réduire la tension fournie au transducteur ultrasonore,
appliquer de manière pulsée la tension fournie au transducteur ultrasonore en le mettant en marche et en l'arrêtant, ou
amener le transducteur ultrasonore à recevoir la tension fournie jusqu'à ce qu'une température ambiante ou une température de transducteur ultrasonore dépasse un seuil actif, puis, à l'atteinte du seuil de température, amener le transducteur ultrasonore à cesser de recevoir la tension fournie jusqu'à ce que la température ambiante ou la température ultrasonore atteigne un seuil de repos.

7. Système (100) selon la revendication 1, dans lequel le microcontrôleur (1108) est programmé pour rechercher le point de résonance en mesurant le courant pilotant le transducteur ultrasonore (1106) lors d'un balayage de la fréquence et de la tension du transducteur ultrasonore.

8. Système (100) selon la revendication 1, dans lequel le microcontrôleur (1108) est programmé pour maintenir ou modifier le point de résonance par au moins l'une des opérations suivantes :
pré-chauffer le sonicateur (1104) en mettant en marche le transducteur ultrasonore (1106) avant que le sonicateur ne soit amené en contact avec la cartouche (120),
pré-charger une force de contact du sonicateur sur la cartouche avant de mettre en marche le transducteur ultrasonore,
faire pulser la force de contact du sonicateur sur la cartouche lors de la mise en marche du transducteur ultrasonore, ou
utiliser une force de contact variable du sonicateur sur la base de l'énergie de transducteur ultrasonore mesurée comme étant consommée afin d'optimiser un transfert d'énergie dans l'échantillon.

9. Système (100) selon la revendication 1, dans lequel le microcontrôleur (1108) est programmé pour alterner entre un mélange ou une agitation de l'échantillon à une fréquence sur la fréquence du point de résonance et en dehors de la fréquence du point de résonance.

10. Système (100) selon la revendication 1, dans lequel le microcontrôleur (1108) est programmé pour déterminer une efficacité de la sonication en effectuant au moins l'une des opérations suivantes :
mesurer une opacité de l'échantillon avant la sonication et comparer l'opacité de l'échantillon mesurée à une valeur d'opacité,
mesurer l'opacité de l'échantillon pendant la sonication et comparer l'opacité de l'échantillon mesurée à la valeur d'opacité,
mesurer l'opacité de l'échantillon après la sonication et comparer l'opacité de l'échantillon mesurée à la valeur d'opacité, ou
déterminer des variations absolues ou relatives de l'opacité de l'échantillon sur la base de valeurs respectivement absolues ou relatives, dans l'opacité de l'échantillon prises avant et après la sonication.

11. Système (100) selon la revendication 10, dans lequel le microcontrôleur (1108) est programmé pour recevoir une mesure de l'opacité de l'échantillon, et délivrer en sortie une alerte d'échec de sonication lorsque la mesure de l'opacité de l'échantillon n'atteint pas ou ne dépasse pas une valeur seuil d'opacité de l'échantillon.

12. Système (100) selon la revendication 1, dans lequel le microcontrôleur (1108) est programmé pour déterminer une dérive du point de résonance en dehors d'une spécification fonctionnelle en
mesurant des variations de courant, de température et de fréquence ; et
en comparant les mesures de variations de courant, de température et de fréquence à un seuil.

13. Système (100) selon la revendication 1, dans lequel le microcontrôleur (1108) est programmé pour déterminer une puissance maximale délivrée à chaque cycle temporel et pour ajuster une fréquence pour compenser une dérive estimée de la fréquence du point de résonance du transducteur ultrasonore (1106), due aux effets d'une force de pression sur la tête de sonde (1102) et à une résonance mécanique de contact.

14. Système (100) selon la revendication 13, dans lequel le microcontrôleur (1108) est en outre programmé pour balayer, au début de chaque cycle d'impulsions, des fréquences de déviation connues, et déterminer une puissance maximale délivrée au moyen d'une boucle proportionnelle-intégrale-dérivée (PID) définie afin de s'ajuster à l'erreur la plus faible sur la base de la résonance inductance-capacité d'un circuit de pilotage.

15. Système (100) selon la revendication 14, dans lequel le microcontrôleur est en outre programmé pour identifier un transducteur ultrasonore (1106) non fonctionnel ou surchargé en mesurant un courant côté haut d'un circuit de pilotage et en comparant le courant côté haut à des données de courant du transducteur ultrasonore.
